# EUROPEAN PATENT APPLICATION

(11) **EP 4 012 021 A1**
(43) Date of publication of application: **15.06.2022**
(21) Application number: 19940758.6
(22) Date of filing: 05.08.2019
(51) Int. Cl.: C12N 5/071, C12N 5/09

(54) **METHOD FOR CULTURING PRIMARY CELLS OF LUNG CANCER SOLID TUMOR AND PRIMARY TUMOR CELLS OF LUNG CANCER PLEURAL EFFUSION, AND SUPPORTING REAGENT**

(71) Applicant: Genex Health Co.,Ltd, Beijing 100195 (CN)
(72) Inventor: YIN, Shenyi, Beijing 101105 (CN); ZHANG, Hanshuo, Beijing 101105 (CN)
(74) Representative: Isarpatent
(86) International application number: PCT/CN2019/099246
(87) International publication number: WO 2021/022440

(57) **Abstract**

The present invention discloses a method for culturing primary cells from solid tumor tissues of lung cancer and primary tumor cells from pleural effusion of lung cancer, and the auxiliary reagents. The present invention provides a method for culturing primary cells from solid tumor tissues of lung cancer and primary tumor cells from pleural effusion of lung cancer and the auxiliary reagents. The core of the technology is as follows: (1) solid tumor of lung cancer are treated by using a mild cell dissociating reagent, and lung cancer cells in pleural effusion are dissociated by a mild method, ensuring the vitality of cancer cells to the greatest extent; (2) a special serum-free medium is prepared, and tumor cells derived from solid tumor tissues of lung cancer are cultured in vitro by using a suspension culture system, ensuring the normal amplification of the cancer cells while eliminating the interference of normal cells to the greatest extent. The primary cell cultures of lung cancer obtained by the method of the present invention can be used for in vitro tests, second-generation sequencing, building of animal models, building of cell lines and the like at various cell levels. It is predictable that such culture method will have a wide application prospect in the fields of research, clinical diagnosis, and treatment of lung cancer.

## Description

### TECHNICAL FIELD

The present invention relates to the technical field of biology, and specifically relates to a method for culturing primary cells from solid tumor of lung cancer and primary tumor cells from pleural effusion of lung cancer and auxiliary reagents.

### BACKGROUND

Lung cancer is a cancer with the highest incidence and mortality in the world, and lung cancer in male has the highest incidence and mortality among all malignant tumors, while the lung cancer in female has the second highest incidence and mortality. Meanwhile, the growth rate of incidence and mortality of lung cancer is the highest among all malignant tumors. It can be said that lung cancer is one of the most threatening malignant tumors to human health and life.

Although scientific research institutions and medical institutions all over the world have invested heavily in the research on the causes, occurrence and development course of lung cancer, humans still know very little about this disease. Lung cancer is a complex disease, its occurrence and development is a dynamic process, involving the interactions of many signaling molecules, and forming a complex molecular regulatory network; and meanwhile, it is also affected by external environmental factors. The causes, occurrence and development course of lung cancer cannot be generalized because of the great individual differences.

Chemotherapy and targeted therapy are the most important treatment methods for patients with advanced lung cancer who missed the opportunity for surgery. include the choice of the first chemotherapy drugs regimen for newly treated patients is particularly important for the control of the patient's disease. With the widespread application of the next-generation sequencing technology, potentially effective drugs can be predicted and found out for only 30%-50% of the patients with lung cancer according to the results of individualized gene sequencing and data mining, and the efficacy of such drugs regimens in killing cancer cells in the actual chemotherapy process cannot be guaranteed.

Traditionally, a lung cancer cell line is used for the research on lung cancer, but it is hard to reflect the true conditions of cancer cells in thousands of different patients with lung cancer, and thus there is a great limitation. The PDX model, which represents the concept of precision medicine, is difficult to overcome the disadvantages of a longer modeling cycle and inability in guiding the clinical treatment. Patients with middle and advanced lung cancer are often complicated by pleural effusion that needs to be excreted in time. Therefore, pleural effusion is a very accessible clinical sample, and exfoliated lung cancer cells can often be found in pleural effusion. It is a trend in the field of lung cancer research and even the field of lung cancer diagnosis and treatment to use cultures of primary cells from solid tumors of lung cancer and cultures of primary tumor cells from pleural effusion of lung cancer as models for individualized and precise research.

The existing technologies for culturing primary tumor cells mainly include 2D culturing, 3D culturing, reprogramming culturing, etc. These methods all face the problems such as extremely long culture cycle, low success rate of culturing, and difficulty in removing miscellaneous cells to varying degrees.

### SUMMARY

To effectively solve the above-mentioned technical problems, the present invention provides a new technology for culturing primary cells from solid tumor of lung cancer and primary tumor cells from pleural effusion of lung cancer and the auxiliary reagents. The core of the technology is as follows: (1) solid tumor of lung cancer are treated with a mild cell dissociating reagent, and lung cancer cells in pleural effusion are dissociated by a mild method to ensure the vitality of cancer cells to the greatest extent; (2) a special serum-free medium is prepared, and tumor cells derived from solid tumor of lung cancer and primary tumor cells from pleural effusion of lung cancer are cultured in vitro by a suspension culture system to ensure the normal amplification of cancer cells while eliminating the interference of normal cells to the greatest extent.

In a first aspect, the present invention claims a medium for culturing primary cells of lung cancer.

The medium for culturing primary cells of lung cancer claimed by the present invention is composed of a dual-antibody P/S (Penicillin-Streptomycin), HEPES, a non-essential amino acid solution, GlutaMax, a human recombinant protein EGF, a human recombinant protein bFGF, a human recombinant protein MSP, Cortisol, B27, ITS-X (Insulin, Transferrin, Selenium, Ethanolamine Solution), Y-27632 and an Advanced DMEM/F12 medium; wherein the final concentration of Penicillin in the dual-antibody P/S is 100-200 U/mL (such as 100 U/mL); the final concentration of Streptomycin in the dual-antibody P/S is 100-200 µg /mL (such as 100 µg/mL); the final concentration of the HEPES is 8-12 mM (such as 10 mM); the final concentration of the non-essential amino acid solution is 0.8-1.2% (such as 1%, % represents a volume percentage); the final concentration of the GlutaMax is 0.8-1.2% (such as 1%, % represents a volume percentage); the final concentration of the human recombinant protein EGF is 10-100 ng/mL; the final concentration of the human recombinant protein bFGF is 10-50 ng/mL; the final concentration of the human recombinant protein MSP is 5-25 ng/mL; the final concentration of the cortisol is 20-50 ng/mL; the final concentration of the B27 is 1.5-2.5% (such as 2%, % represents a volume percentage); the final concentration of the ITS-X is 0.8-1.2% (such as 1%, % represents a volume percentage); the final concentration of the Y-27632 is 5-20 µM; the rest is the Advanced DMEM/F12 medium.

Further, the solvent for the non-essential amino acid solution is water, and solutes and their concentrations are as follows: 10 mM of glycine; 10 mM of L-alanine; 10 mM of L-asparagine; 10 mM of L-aspartic acid; 10 mM of L-glutamine acid; 10 mM of L-proline; and 10 mM of L-serine. The B27 is "B-27^{™} Supplement (50X), minus vitamin A" (such as Gibco#12587010, or other products with the same composition), wherein the "B-27^{™} Supplement (50X), minus vitamin A" contains biotin, DL Alpha Tocopherol Acetate, DL Alpha Tocopherol, BSA (fatty acid free Fraction V), Catalase, Human Recombinant Insulin, Human Transferrin, Superoxide Dismutase, Corticosterone, D-Galactose, Ethanolamine HCl, Glutathione (reduced), L-Carnitine HCl, Linoleic Acid, Linolenic Acid, Progesterone, Putrescine 2HCl, Sodium Selenite and T3 (triodo-I-thyronine). The solvent of the ITS-X is EBSS solution (Earle's balanced salt solution), and the solutes and their concentrations are as follows: 1 g/L Insulin; 0.55 g/L Transferrin; 0.00067 g/L Sodium Selenite; and 0.2 g/L Ethanolamine. The GlutaMAX is an advanced cell culture additive that can directly replace L-Glutamine in a cell culture medium. The GlutaMAX is "GlutaMAX^{™} Supplement" (such as Gibco#35050061, or other products with the same composition). The "GlutaMAX^{™} Supplement" with L-Alanyl-L-Glutamine as its component is a substitute for L-Glutamine, at a concentration of 200 nM, and the solvent is 0.85% NaCl solution. The Y-27632 is "Y-27632 dihydrochloride (an ATP-competitive ROCK-I and ROCK-II inhibitor, Ki is 220 nM and 300 nM respectively)" (such as MCE#129830-38-2, or other products with the same composition).

In an embodiment of the present invention, the brand article No. of the dual-antibody P/S (penicillin-streptomycin) is Gibco#15140122; the brand article No. of the HEPES is Gibco#15630080; the brand article No. of the non-essential amino acid solution is Gibco#1 1140-050; the brand article No. of the GlutaMAX is Gibco#35050061; the brand article No. of the human recombinant protein EGF is Peprotech AF-100-15-100; the brand article No. of the human recombinant protein bFGF is Peprotech AF-100-18B-50; the brand article No. of the human recombinant protein MSP is R&D#352-MS-050; the brand article No. of the cortisol is Sigma#H0888; the brand article No. of the B27 is Gibco#12587010; the brand article No. of the ITS is Gibco#51500056; the brand article No. of the Y-27632 is MCE#129830-38-2; and the brand article No. of the Advanced DMEM/F12 medium is Gibco#12634010.

Further, the medium for culturing primary cells of lung cancer can exist in two forms:
First, the medium for culturing primary cells of lung cancer is a solution prepared by mixing the dual-antibody P/S, the HEPES, the non-essential amino acid solution, the GlutaMax, the human recombinant protein EGF, the human recombinant protein bFGF, the human recombinant protein MSP, the cortisol, the B27, the ITS-X, the Y-27632 and the advanced DMEM/F12 medium;

The prepared culture medium needs to be filtered and sterilized by a 0.22 µM syringe-driven strainer (Millipore SLGP033RS) of, and can be stored at 4 °C for two weeks.

Second, each component in the medium for culturing primary cells of lung cancer exist separately, and can be prepared according to a formula before use.

Even further, the human recombinant protein EGF, the human recombinant protein bFGF, the human recombinant protein MSP, the cortisol and the Y-27632 exist in a form of stock solution (mother solution) (and can be stored at -80 °C for a long time); and specifically, they can be 1,000× stock solutions (mother solutions).

A 1,000× human recombinant protein EGF stock solution is composed of the human recombinant protein EGF, BSA and PBS, wherein the final concentration of the human recombinant protein EGF is 20 µg/mL, the final concentration of the BSA is 0.01 g/mL, and the rest is PBS.

A 1,000× human recombinant protein bFGF stock solution is composed of human recombinant protein bFGF, BSA and PBS, wherein the final concentration of the human recombinant protein bFGF is 20 µg/mL, the final concentration of the BSA is 0.01 g/mL, and the rest is PBS.

A 1,000× human recombinant protein MSP stock solution is composed of human recombinant protein MSP, BSA and PBS, wherein the final concentration of the human recombinant protein MSP is 20 µg/mL, the final concentration of the BSA is 0.01 g/mL, and the rest is PBS.

Among the above three 1,000× stock solutions, the BSA can exist in a form of 100× stock solution (mother solution) (prepared just before use), and specifically, it is composed of BSA and PBS, wherein the final concentration of the BSA (Sigma#A1933) is 0.1 g/mL, and the rest is PBS.

Besides, the stock solution of 1,000× cortisol is composed of cortisol, absolute ethanol and ultrapure water, wherein the final concentration of the cortisol is 25 µg/mL, the final concentration of the absolute ethanol is 5% (volume percentage), and the rest is ultrapure water.

A 1,000× Y-27632 stock solution is composed of Y-27632 and ultrapure water, in which the final concentration of the Y-27632 is 10 mM, and the rest is ultrapure water.

In a second aspect, the present invention claims a reagent set for culturing primary cells of lung cancer.

The reagent set claimed by the present invention can be any of the followings:
(A1) Consisting of the culture medium in the first aspect and all or part of the following components: a sample dissociation solution, a sample preservation solution and a sample washing solution;
(A2) Consisting of the culture medium in the first aspect and a cell isolation buffer;
(A3) Consisting of (A1) and all or part of the following reagents: a cell digestion solution, a digestion termination solution, and a cell cryopreservation solution; and
(A4) Consisting of (A2) and all or part of the following reagents: a cell digestion solution, a digestion termination solution, and a cell cryopreservation solution.

The sample dissociation solution is composed of collagenase I, collagenase IV and PBS; wherein the final concentration of the collagenase I in the sample dissociation solution is 150-250 U/mL (such as 200 U/mL); the final concentration of the collagenase IV in the sample dissociation solution is 150-250 U/mL (such as 200 U/mL); and the rest is PBS.

Wherein a unit U of the collagenase (the collagenase I or the collagenase IV) is defined by the enzyme activity of the proteases: under the conditions of 37°C and a pH value of 7.5, when the collagenase (the collagenase I or the collagenase IV) is treated by 1 U of proteases for 5 hours, 1 µmol of L-leucine can be released.

In an embodiment of the present invention, the brand article No. of the collagenase I is Gibco#17100-017; the brand article No. of the collagenase IV is Gibco#17104-019; and the brand article No. of the PBS is Gibco#21-040-CVR.

Wherein the sample preservation solution is composed of fetal bovine serum, dual-antibody P/S, HEPES and HBSS (Hank's balanced salt solution); wherein the final concentration of the fetal bovine serum in the sample preservation solution is 1-5% (such as 2%, % represents a volume percentage); the final concentration of the penicillin of the dual-antibody P/S in the sample preservation solution is 100-200 U/mL (such as 100 U/mL); the final concentration of the streptomycin of the dual-antibody P/S in the sample preservation solution is 100-200 µg/mL (such as 100 µg/mL); the final concentration of the HEPES in the sample preservation solution is 8-12 mM (such as 10 mM); and the rest is HBSS.

In an embodiment of the present invention, the brand article No. of the fetal bovine serum is Gibco#16000-044; the brand article No. of the dual-antibody P/S is Gibco#15140122; the brand article No. of the HEPES is Gibco# 15630080; and the brand article No. of the HBSS is Gibco# 14170161.

The sample washing solution is composed of dual-antibody P/S and PBS; wherein the final concentration of the penicillin of the dual-antibody P/S in the sample washing solution is 100-200 U/mL (such as 100 U/mL); the final concentration of the streptomycin of the dual-antibody P/S in the sample washing solution is 100-200 µg/mL (such as 100µg/mL); and the rest is PBS.

In an embodiment of the present invention, the brand article No. of the dual-antibody P/S is Gibco#15140122; and the brand article No. of the PBS is Gibco#21-040-CVR.

The cell isolation buffer is composed of dual-antibody P/S (penicillin-streptomycin), heparin sodium and PBS; wherein the final concentration of the penicillin in the dual-antibody P/S is 100-200 U/mL (such as 100 U/mL); the final concentration of the streptomycin in the dual-antibody P/S is 100-200 µg/mL (such as 100 µg/mL); the final concentration of the heparin sodium is 10 IU/mL; and the rest is PBS.

In an embodiment of the present invention, the brand article No. of the dual-antibody P/S is Gibco#15140122; the brand article No. of the heparin sodium is Solarbio#H8270; the brand article No. of the PBS is Gibco#21-040-CVR.

The cell digestion solution is composed of the followings: each 10 mL of the cell digestion solution contains 4-6 mL (such as 5 mL) of Accutase, EDTA with a final concentration of 5 mM (such as 10 µL of 0.5 M EDTA), 1.5-2.5 mL (such as 2 mL) of TrypLE Express and the rest is PBS.

Further, the Accutase is "StemPro^{™} Accutase^{™} Cell Dissociation Reagent" (such as Gibco#A11105-01, or other products with the same composition). The Accutase is a single-component enzyme which is dissolved in D-PBS and 0.5 mM EDTA solution. The TrypLE Express is "TrypLE^{™} Express Enzyme (IX), no phenol red" (such as Gibco#12604013, or other products with the same composition). The "TrypLE^{™} Express Enzyme (IX), no phenol red" contains 200 mg/L KCl, 200 mg/L KH₂PO₄, 8,000 mg/L NaCl, 2160 mg/L Na₂HPO₄·7H₂O and 457.6 mg/L EDTA; and it also contains recombinant protease.

In an embodiment of the present invention, the brand article No. of the Accutase is Gibco#A11105-01; the brand article No. of the 0.5 M EDTA is Invitrogen#AM9261; the brand article No. of the TrypLE Express is Gibco#12604013; and the brand article No. of the PBS is Gibco#21-040-CVR.

The digestion termination solution is composed of fetal bovine serum, dual-antibody P/S and a DMEM medium; wherein the final concentration of the fetal bovine serum in the digestion termination solution is 8-12% (such as 10%, % represents a volume percentage); the final concentration of the penicillin of the dual-antibody P/S in the digestion termination solution is 100-200 U/mL (such as 100 U/mL); the final concentration of the streptomycin of the dual-antibody P/S in the digestion termination solution is 100-200 µg/mL (such as 100 µg /mL); and the rest is the DMEM medium.

In an embodiment of the present invention, the brand article No. of the fetal bovine serum is Gibco#16000-044; the brand article No. of the dual-antibody P/S is Gibco#15140122; and the brand article No. of the DMEM medium is Gibco#11965-092.

The cell cryopreservation solution is composed of Advanced DMEM/F12 medium, DMSO and 1% methylcellulose solution; wherein the volume ratio of the Advanced DMEM/F12 medium, the DMSO and the 1% methylcellulose solution is 20:2:(0.8-1.2), such as 20:2:1; and the 1% methylcellulose solution is an aqueous solution of methylcellulose with a concentration of 1 g/100 ml.

In an embodiment of the present invention, the brand article No. of the Advanced DMEM/F12 medium is Gibco#12634010; the brand article No. of the DMSO is Sigma#D2438; and the brand article No. of the methylcellulose is Sigma#M7027.

The sample preservation solution can be used for temporarily preserving the extracted samples, and can maintain the viability of cells in the samples within a short period of time after the samples have been extracted. The sample preservation solution can be stored for 1 month at 4 °C after preparation.

The sample washing solution can be used for washing and disinfecting samples. The sample washing solution needs to be prepared just before use.

The sample dissociation solution can be used for samples dissociation, and can dissociate primary cells from solid tumor tissues of lung cancer in the sample from the tissue. The sample dissociation solution needs to be prepared just before use. The collagenase I and collagenase IV can be preserved in a form of stock solution (mother solution) at -20 °C for a long period, specifically 10× stock solution (mother solution). The 10× stock solution of collagenase I is composed of the collagenase I and PBS, wherein the final concentration of the collagenase I is 2,000 U/mL and the rest is PBS. The 10× stock solution of collagenase IV is composed of the collagenase IV and PBS; wherein the final concentration of the collagenase IV is 2,000 U/mL; and the rest is PBS. The enzyme activities of the collagenase I and the collagenase IV are defined above.

The cell isolation buffer is used for suspending cells in pleural fluid so as to prepare for the density gradient separation of the cells. The cell isolation buffer can be stored at 4 °C for 1 month after preparation.

The cell digestion solution can be used for digesting and passaging cell masses, and can digest a tumor mass of lung cancer into single cells. The cell digestion solution needs to be prepared just before use.

The digestion termination solution can be used to terminate a process of sample dissociation or cell digestion. The digestion termination solution can be stored at 4 °C for 1 month after preparation.

The cell cryopreservation solution needs to be prepared just before use, wherein the 1% methylcellulose solution can be stored at 4 °C for a long period.

In a third aspect, the present invention claims any of the following applications:
(B1) an application of the culture medium in the first aspect in culturing primary cells of lung cancer;
(B2) an application of the reagent set previously described in (A1) or (A3) in the second aspect in culturing primary cells from solid tumor of lung cancer;
(B3) an application of the reagent set previously described in (A2) or (A4) in the second aspect in culturing primary tumor cells from pleural effusion of lung cancer.

In a fourth aspect, the present invention claims a method for culturing primary cells of lung cancer.

The method for culturing primary cells of lung cancer claimed by the present invention is either method A or method B:
Method A: a method for culturing primary cells from solid tumor of lung cancer, including the following steps:
   (a1) dissociating solid tumor tissues of lung cancer with the sample dissociation solution previously described in the second aspect, to obtain primary cells from solid tumor of lung cancer;
   (a2) suspension-culturing the dissociated primary cells from solid tumor of lung cancer in step (a1) with the culture medium previously described in the first aspect.
Method B: a method for culturing primary tumor cells from pleural effusion of lung cancer, including the following steps::
   (b1) separating and obtaining primary tumor cells from pleural effusion of lung cancer;
   (b2) suspension-culturing the separated primary tumor cells from pleural effusion of lung cancer in step (b1) with the culture medium of claim 1.

Further, in step (a1), the solid tumor tissues of lung cancer can be dissociated with the sample dissociation solution according to a method including the following steps: according to the dosage of 0.1-0.3 mL (such as 0.1 mL) of sample dissociation solution per 1 mg of tissue, treating the solid tumor tissues of lung cancer, which were cut up (e.g. cut into small pieces of 0.8-1.2 mm³), with the sample dissociation solution preheated to 37°C in advance, and dissociating the sample at 37°C for 15 minutes to 3 hours; observing the dissociation of the sample under a microscope every 15 minutes until a large number of individual cells are observed.

Further, in step (b1), the primary tumor cells from pleural effusion of lung cancer can be separated from pleural effusion of lung cancer according to a method including the following steps: suspending the cells in the pleural effusion of lung cancer with the cell isolation buffer previously described in the second aspect, and then obtaining the primary tumor cells from pleural effusion of lung cancer by density gradient centrifugation (using a Ficoll lymphocyte separation medium).

Further, in step (a2), the primary cells from solid tumor of lung cancer can be suspension-cultured with the culture medium for the primary cells from solid tumor of lung cancer according to a method including the following steps: using a cell culture vessel M, suspension-culturing the primary cells from solid tumor of lung cancer with the culture medium, under the condition of 37°C and 5% CO2, and replacing the culture medium every 2-4 days (such as 3 days) until the cell masses with a diameter of 80-120 µm (such as 100 µm) are formed.

Further, in the step (b2), the primary tumor cells from pleural effusion of lung cancer can be suspension-cultured with the culture medium according to a method including the following steps: using a cell culture vessel M, suspension-culturing the primary tumor cells from pleural effusion of lung cancer with the culture medium, under the condition of 37°C and 5% CO₂, and replacing the culture medium every 2-4 days (such as 3 days) until the cell masses with a diameter of 80-120 µm (such as 100 µm) are formed.

Wherein the initial inoculation density can be 10⁵ cells/cm² vessel bottom area; taking a 6-well plate as an example, and cells are planked at a density of 10⁶ cells per well.

Wherein the cell culture vessel M can be any of the followings: (I) a cell culture vessel made of polystyrene, a cell culture vessel made of polycarbonate, a cell culture vessel made of polymethylmethacrylate, a cell culture vessel made of COC resin, a cell culture vessel made of cycloolefin polymer or a cell culture vessel with a low adsorption surface; (II) a cell culture vessel after CYTOP modification of the cell culture vessel in (I).

Further, the cell culture vessel is a cell culture dish, a cell culture well plate or a microplate chip for cell culture.

In (II), the cell culture vessel in (I) can be CYTOP-modified according to a method including the following steps: performing pure oxygen etching on the cell culture vessel in (I) at an etching power of 20 W for 3 minutes; then covering the surface of the cell culture vessel with 1% CYTOP solution, and drying the 1% CYTOP solution in the air to complete CYTOP modification.

Wherein the composition of the 1% CYTOP solution is as follows: each 100 mL of the 1% CYTOP solution contains 1 mL of CYTOP, and the rest is fluorocarbon oil.

Further, before step (a1), the following steps for predissociation treatment of the solid tumor tissues of lung cancer can also be included: washing the surface of the solid tumor tissue sample of lung cancer with 70-75% ethanol (by volume, such as 75%) for 10-30 seconds; washing the solid tumor tissue sample of lung cancer for 5-10 times (such as 5 times) with the sample washing solution, and washing the solid tumor tissue sample of lung cancer for 5-10 times (such as 5 times) with sterile PBS solution; then removing impurities, connective tissues, adipose tissues, necrotic tissues and other components affecting primary cell culture from the solid tumor tissue sample of lung cancer.

The steps of predissociation treatment of the solid tumor tissues of lung cancer need to be performed on ice, and all the steps need to be completed within 10 minutes.

Further, the ex vivo time of the solid tumor tissue sample of lung cancer subjected to the predissociation treatment needs to be within 2 hours, and the solid tumor tissue sample of lung cancer must be preserved in the sample preservation solution before the predissociation treatment.

Further, in step (a1), the following steps can also be included after the dissociation treatment of the solid tumor tissue of lung cancer with the sample dissociation solution: terminating the dissociation reaction with 8-15 times (such as 10 times) the volume of the digestion termination solution, and collecting the cell suspension; filtering the cell suspension with a 100 µm or 40µm sterile cell strainer to remove tissue debris and adherent cells; centrifuging at 800-1,000 g (such as 800 g) at room temperature for 10-15 minutes (such as 10 minutes), and discarding the supernatant; resuspending cells with 3-5 mL (such as 5mL) sterile PBS; re-centrifuging at 800-1,000 g (such as 800 g) at room temperature for 10-15 minutes (such as 10 minutes), and discarding the supernatant; then, resuspending the cell precipitation with the culture medium for primary cells from solid tumor of lung cancer, observing the cell state under a microscope, and counting the cells.

Further, before step (b1), the steps of pre-separation treatment of the pleural effusion samples of lung cancer can also be included: removing impurities, blood clots and other components that affect the cell density gradient separation in the pleural effusion samples of lung cancer.

Further, before step (a2), the following step can also be included: passaging the primary cells of solid tumor of lung cancer when the primary cells of solid tumor of lung cancer forms a mass with a diameter of 80-120 µm (such as 100 µm).

Further, in step (b2), the following step can also be included: passaging the primary tumor cells of the pleural effusion samples of lung cancer when the primary tumor cells of the pleural effusion samples of lung cancer forms a mass with a diameter of 80-120 µm.

Wherein the cell digestion solution for the passage is the cell digestion solution previously described in the second aspect.

Wherein the digestion termination solution for the passage is the digestion termination solution previously described in the second aspect.

Further, the dissociation temperature used in the passage is 37 °C.

More specifically, the passaging steps are as follows: collecting the cell masses to be passaged, washing the cell masses with sterile PBS solution after centrifugation, resuspending the cell masses with the cell digestion solution after re-centrifugation, digesting the cell masses at 37°C until all the cell masses are digested into individual cells, terminating the digestion reaction with the digestion termination solution (the dosage may be 5-10 times the volume, such as 10 times the volume), and collecting the cell suspension; after centrifugation, resuspending the cell precipitation with the culture medium previously described in the first aspect, counting, and then suspension-culturing the cells in the cell culture vessel M previously described (the initial inoculation density can be 10⁵ cells/cm² vessel bottom area; taking a 6-well plate as an example, and cells are planked at a density of 10⁶ cells per well) under the condition of 37°C and 5% CO₂. All the centrifugations in the above passage steps can be specifically centrifugations at 800-1,000 g (such as 800 g) at room temperature for 10-20 minutes (such as 10 minutes).

Further, the method can also include a step of cryopreserving and/or resuscitating the primary cells from solid tumor of lung cancer or the primary tumor cells from pleural effusion of lung cancer after 2-3 passages and amplifications. The cell cryopreservation solution used for the cryopreservation is the cell cryopreservation solution previously described in the second aspect.

Further, the specific steps of performing the cryopreservation are as follows: collecting the cell masses to be cryopreserved, washing the cell masses with sterile PBS solution after centrifugation, re-centrifuging, resuspending the cell masses with the cell digestion solution, digesting at 37 °C until all the cell masses are digested into individual cells, terminating the digestion reaction with the digestion termination solution (the dosage may be 5-10 times, such as 10 times the volume), and collecting the cell suspension; after centrifugation, resuspending the cell precipitation with the cell cryopreservation solution at a density of 0.5-2×10⁶/mL (such as 10⁶/mL), cytopreserving in a gradient cooling box overnight and transferring into liquid nitrogen for a long-term preservation. All the centrifugations in the above cryopreserving steps can be ones at 800-1,000 g (such as 800 g) at room temperature for 10-20 minutes (such as 10 minutes).

Even further, the specific steps of performing the resuscitation are as follows: removing a cryopreserving tube containing the cells to be resuscitated from liquid nitrogen, and quickly melting the cells in sterile water at 37-39°C (such as 37°C); after centrifugation (such as at 800-1,000 g, e.g. centrifugation at 800 g at room temperature for 5-10 minutes, such as 10 minutes), resuspending the cell precipitation with the culture medium previously described in the first aspect, then suspension-culturing the cells in the cell culture vessel M previously described (the initial inoculation density can be 10⁵ cells/cm² vessel bottom area), and cryopreserving the cells (10⁶ cells) in each tube to a 3.5 cm culture dish under the condition of 37°C and 5% CO₂.

In a fifth aspect, the present invention claims any of the following reagents:
(C1) the sample dissociation solution of solid tumor tissues of lung cancer, which is the sample dissociation solution previously described in the second aspect;
(C2) the sample preservation solution of solid tumor tissues of lung cancer, which is the sample preservation solution previously described in the second aspect;
(C3) the cell isolation buffer for cells from pleural effusion of lung cancer, which is the cell isolation buffer previously described in the second aspect;
(C4) the cell digestion solution for primary cells of lung cancer, which is the cell digestion solution previously described in the second aspect.

In a sixth aspect, the present invention claims any of the following applications:
(D1) an application of the sample dissociation solution previously described in (C1) in the fifth aspect in dissociating the primary cells from solid tumor tissues of lung cancer from the solid tumor tissues of lung cancer.
(D2) an application of the sample preservation solution previously described in (C2) in the fifth aspect in preserving the solid tumor tissues of lung cancer.
(D3) an application of the cell isolation buffer previously described in (C3) in the fifth aspect in isolation of the primary tumor cells in pleural effusion of lung cancer from the pleural effusion of lung cancer.
(D4) an application of the cell digestion solution previously described in (C4) in the first aspect in passaging primary cells of lung cancer.

In a seventh aspect, the present invention claims any of the following methods:
(E1) a method for dissociating primary cells of solid tumor of lung cancer from the solid tumor tissues of lung cancer, including the step (a1) in the method previously described in the fourth aspect.
(E2) a method for preserving solid tumor tissue of lung cancer, including the following steps: preserving the solid tumor tissues of lung cancer just detached in the sample preservation solution previously described in the second aspect for no more than 2 hours.
(E3) a method for separating primary tumor cells in pleural effusion of lung cancer from the pleural effusion of lung cancer including the step (b1) in the method previously described in the fourth aspect.
(E4) a method for passaging primary cells of lung cancer, including the following steps: passaging the primary cells of lung cancer when the primary cells of lung cancer forms a masse with a diameter of 80-120 µm; the cell digestion solution used for passaging is the cell digestion solution previously described in the second aspect; the digestion termination solution used for passaging is the digestion termination solution previously described in the second aspect.

In the above aspects, the lung cancer can be primary lung cancer. The pathological type is non-small-cell lung cancer or small cell lung cancer. The pathological staging is stage II, III or IV. Furthermore, samples at stage II or III are preferred to separate primary cells of lung cancer from solid tumor tissues of lung cancer, and samples obtained by separating primary cells of lung cancer from pleural effusion of lung cancer are samples at stage IV.

Further, the non-small-cell lung cancer can be adenocarcinoma of non-small-cell lung cancer or squamous cell carcinoma of non-small-cell lung cancer.

In the above-mentioned aspects, the primary cells of lung cancer can be primary cells from solid tumor of lung cancer or primary tumor cells from pleural effusion of lung cancer.

In the above aspects, the primary cells of lung cancer can be isolated from surgical samples (of a solid tumor tissue),a puncture sample (of a solid tumor tissue) or a pleural effusion sample of a patient with lung cancer. Wherein the solid tumor tissue specimen of lung cancer obtained from the surgical sample preferably weighs more than 20 mg. The volume of the pleural effusion sample is preferably not less than 50 mL. The number of puncture samples is not less than 2.

In the present invention, all the above described PBS may be 1× PBS, with pH 7.3-7.5. The specific composition is as follows: the solvent is water, and the solute and concentration are as follows: KH2PO4 144 mg/L, NaCl 9,000 mg/L and Na₂HPO₄·7H₂O 795 mg/L.

### BRIEF DESCRIPTION OF THE DRAWINGS

FIG. 1 illustrates the single cells obtained from lung cancer tissues after treatment. The scale is 200 µm, 10× magnification.
FIG. 2 illustrates the cell masses obtained from lung cancer tissues after primary culturing. The scale is 100 µm, 10× magnification.
FIG. 3 illustrates a HE staining image of lung cancer cells obtained from lung cancer tissues after primary culturing. The scale is 50 µm, 40x magnification.
FIG. 4 illustrates an immunofluorescence staining image of cancer cell masses obtained from lung cancer tissues after primary culturing.
FIG. 5 illustrates a copy number variation (CNV) analysis based on the sequencing results, showing that the copy number variation of all generations of primary cell cultures of lung cancer (P1, P2, P3 and P4) is highly consistent with that of the primary tumor tissue (Tumor) of lung cancer.
FIG. 6 illustrates the single cells obtained from a pleural effusion sample of lung cancer. The scale is 200 µm.
FIG. 7 illustrates the cell masses obtained from primary tumor cells in a pleural effusion sample of lung cancer after culturing. The scale is 200 µm.
FIG. 8 illustrates a HE staining image of lung cancer cells obtained from a pleural effusion sample of lung cancer after primary culturing. The scale is 100 µm.
FIG. 9 illustrates an immunofluorescence staining image of cancer cell masses obtained from a pleural effusion sample of lung cancer after primary culturing.
FIG. 10 illustrates a copy number variation (CNV) analysis based on the sequencing results, showing that the copy number variation of all generations of primary cell cultures of lung cancer (P1, P2, P3, P4 and P5) is highly consistent with that of the tumor cells in pleural effusion of lung cancer.
FIG. 11 illustrates the results of an in vitro chemosensitivity assay on the primary cells from pleural effusion of lung cancer cultured by using the present invention.
FIG. 12 illustrates a design view of a microplate chip of the present invention.

### DETAILED DESCRIPTION OF THE EMBODIMENTS

The following embodiments are intended for a better understanding of the present invention, but not limiting the present invention. Unless otherwise specified, all the experimental methods in the following embodiments are conventional methods. Unless otherwise specified, all the test materials used in the following embodiments are available from the conventional biochemical stores.

### Embodiment 1. Preparing reagents for culturing primary cells from solid tumor of lung cancer

### 1. Sample preservation solution (100 mL)

The specific formula of sample preservation solution (100 mL) is shown in Table 1.

**Table 1 Sample Preservation Solution (100 mL)**

| Reagent | Brand Article No. | Dosage | Final Concentration |
|---|---|---|---|
| Fetal bovine serum | Gibco#16000-044 | 2 mL | 2% |
| Dual-antibody | Gibco#15140122 | 1 mL | 1% |
| P/S | | | |
| HEPES | Gibco# 15630080 | 1 mL | 10 mM |
| HBSS | Gibco# 14170161 | Added to 100 | |
| | | mL | |

After being prepared, the sample preservation solution was sub-packaged in 15 mL centrifuge tubes, and each tube was filled with 5 mL. The sample preservation solution sub-packaged in the centrifuge tubes can be stored at 4°C for 1 month.

### 2. Sample washing solution (100 mL)

The specific formula of sample washing solution (100 mL) is shown in Table 2.

**Table 2 Sample Washing Solution (100 mL)**

| Reagent | Brand Article No. | Dosage | Final Concentration |
|---|---|---|---|
| Dual-antibody | Gibco#15140122 | 1 mL | 1% |
| P/S | | | |
| PBS | Gibco#21-040-CVR | Added to 100 mL | |

The sample washing solution needs to be prepared just before use.

### 3. Sample dissociation solution (10 mL)

The specific formula of sample dissociation solution (10 mL) is shown in Table 3.

**Table 3 Sample Dissociation Solution (10 mL)**

| Reagent | Brand Article No. | Dosage | Final Concentration |
|---|---|---|---|
| 10× collagenase I | 10× stock solution | 1 mL | 200 U/mL |
| 10× collagenase IV | 10× stock solution | 1 mL | 200 U/mL |
| PBS | Gibco#21-040-CVR | Added to 10 | |
| | | mL | |

| | | | |
|---|---|---|---|
| Note: The sample dissociation solution needs to be prepared just before use. | | | |

In Table 3, the preparation of collagenase stock solution is shown in Tables 4 and 5.

**Table 4 10× Collagenase I Stock Solution (100 mL)**

| Reagent | Brand Article No. | Dosage | Final Concentration |
|---|---|---|---|
| Collagenase I | Gibco #17100-017 | 1 g | 2,000 U/mL |
| PBS | Gibco#21-040-CVR | Added to 100 | |
| | | mL | |

After being prepared, the 10× collagenase I stock solution was sub-packaged in 1.5 mL sterile centrifuge tubes, and each tube was filled with 1 mL. The stock solution can be stored at -20°C for a long term.

**Table 5 10× Collagenase IV Stock Solution (100 mL)**

| Reagent | Brand Article No. | Dosage | Final Concentration |
|---|---|---|---|
| Collagenase IV | Gibco #17104-019 | 1g | 2,000 U/mL |
| PBS | Gibco#21-040-CVR | Added to 100 mL | |

After being prepared, the 10× collagenase IV stock solution was sub-packaged in 1.5 mL sterile centrifuge tubes, and each tube was filled with 1 mL. The stock solution can be stored at -20°C for a long term.

In Tables 4 and 5, the unit U of the collagenase (the collagenase I or the collagenase IV) is defined by the enzyme activity of protease: 1 µmol of L-leucine can be released by treating the collagenase (the collagenase I or the collagenase IV) with 1 U of protease for 5 hours at 37°C and pH 7.5.

### 4. Cell digestion solution (10 mL)

The specific formula of cell digestion solution (10 mL) is shown in Table 6.

**Table 6 Cell Digestion Solution (10 mL)**

| Reagent | Brand Article No. | Dosage | Final Concentration |
|---|---|---|---|
| Accutase | Gibco#A11105-01 | 5 mL | |
| 0.5M EDTA | Invitrogen#AM9261 | 10 µL | 5 mM |
| TrypLE^{™} Express | Gibco#12604013 | 2 mL | |
| PBS | Gibco#21-040-CVR | Added to 10 mL | |

The cell digestion solution needs to be prepared just before use.

### 5. Digestion termination solution (100 mL)

The specific formula of digestion termination solution (100 mL) is shown in Table 7.

**Table 7 Digestion Termination Solution (100 mL)**

| Reagent | Brand Article No. | Dosage | Final Concentration |
|---|---|---|---|
| Fetal bovine serum | Gibco#16000-044 | 10 mL | 10% |
| Dual-antibody P/S | Gibco#15140122 | 1 mL | 1% |
| DMEM culture | Gibco#11965-092 | Added to 100 mL | |
| medium | | | |

The prepared digestion termination solution can be stored for 1 month at 4 °C.

### 6. Culture medium for primary cells from solid tumor tissues of lung cancer (100 mL)

The specific formula of the culture medium for primary cells from solid tumor tissues of lung cancer (100 mL) is shown in Table 8.

**Table 8 Culture Medium for Primary Cells from Solid Tumor Tissues of Lung Cancer (100 mL)**

| Reagent | Brand Article No. | Dosage | Final Concentration |
|---|---|---|---|
| Dual-antibody P/S | Gibco#15140122 | 1 mL | 1% |
| HEPES | Gibco#15630080 | 1 mL | 10 mM |
| Non-essential amino acid solution | Gibco#1 1140-050 | 1 mL | 1% |
| GlutaMax | Gibco#35050061 | 1 mL | 1% |
| 1,000× human recombinant protein EGF | 1,000× stock solution | 50-500 µL | 10-100 ng/mL |
| 1,000× human recombinant protein bFGF | 1,000× stock solution | 50-250 µL | 10-50 ng/mL |
| 1,000× human | 1,000× stock | 25-125 µL | 5-25 ng/mL |
| recombinant protein MSP | solution | | |
| 1,000× cortisol | 1,000× stock solution | 80-200 µL | 20-50 ng/mL |
| B27 | Gibco#12587010 | 2 mL | 2% |
| ITS-X | Gibco#51500056 | 1 mL | 1% |
| 1,000× Y-27632 | 1,000× stock solution | 50-200 µL | 5-20 µM |
| Advanced DMEM/F12 culture medium | Gibco#12634010 | Added to 100 mL | |

After being prepared, the culture medium for primary cells from solid tumor of lung cancer was filtered and sterilized with a 0.22 µM syringe-driven strainer (Millipore SLGP033RS). The culture medium can be stored at 4°C for two weeks.

In Table 8, the preparation of human recombinant protein stock solution is shown in Table 9-Table 12, the preparation of cortisol stock solution is shown in Table 13, and the preparation of Y-27632 stock solution is shown in Table 14.

**Table 9 100× BSA Solution (1 mL)**

| Reagent | Brand Article No. | Dosage | Final Concentration |
|---|---|---|---|
| BSA PBS | Sigma#A1933 Gibco#21-040-CVR | 0.1 g Added to 1 mL | 0.1 g/mL |
| 100× BSA solution needs to be prepared just before use. | | | |
| Table 10 1,000× Human Recombinant Protein EGF Stock Solution (5 mL) | | | |

| Reagent | Brand Article No. | Dosage | Final Concentration |
|---|---|---|---|
| Human recombinant protein EGF | Peprotech AF-100-15-100 | 100 µg | 20 µg/mL |
| 100× BSA solution PBS | Gibco#21-040-CVR | 500 µL Added to 5 mL | 0.01 g/mL |

After being prepared, the 1,000× human recombinant protein EGF stock solution was sub-packaged in 1.5 mL sterile centrifuge tubes. The stock solution can be stored at -80°C for a long term.

**Table 11 1,000× Human Recombinant Protein bFGF Stock Solution (2.5 mL)**

| Reagent | Brand Article No. | Dosage | Final Concentration |
|---|---|---|---|
| Human recombinant protein bFGF | Peprotech AF-100-18B-50 | 50 µg | 20 µg/mL |
| 100× BSA solution | | 250 µL | 0.01 g/mL |
| PBS | Gibco#21-040-CVR | Added to 2.5 mL | |

After being prepared, the 1,000× human recombinant protein bFGF stock solution was sub-packaged in 1.5 mL sterile centrifuge tubes. The stock solution can be stored at -80°C for a long term.

**Table 12 1,000× Human Recombinant Protein MSP Stock Solution (2.5 mL)**

| Reagent | Brand Article No. | Dosage | Final Concentration |
|---|---|---|---|
| Human recombinant | R&D#352-MS-050 | 50 µg | 20 µg/mL |
| protein MSP 100× BSA solution | | 250 µL | 0.01 g/mL |
| PBS | Gibco#21-040-CVR | Added to 2.5 mL | |

After being prepared, the 1,000× human recombinant protein MSP stock solution was sub-packaged in 1.5 mL sterile centrifuge tubes. The stock solution can be stored at -80°C for a long term.

**Table 13 1,000× Cortisol Stock Solution (100 mL)**

| Reagent | Brand Article No. | Dosage | Final Concentration |
|---|---|---|---|
| Cortisol | Sigma#H0888 | 2.5 g | 25 µg/mL |
| Absolute ethyl alcohol | Sigma#E7023 | 5 mL | 5% |
| Ultrapure water | | Added to 100 mL | |

After being prepared, the 1,000× cortisol stock solution was sub-packaged in 1.5 mL sterile centrifuge tubes. The stock solution can be stored at -80°C for a long term.

**Table 14 1,000×Y-27632 Stock Solution (3.125mL)**

| Reagent | Brand Article No. | Dosage | Final Concentration |
|---|---|---|---|
| Y-27632 | MCE#129830-38-2 | 10 g | 10 mM |
| Ultrapure water | | Added to 3.125 mL | |

After being prepared, the 1,000×Y-27632 stock solution was sub-packaged in 0.5 mL sterile centrifuge tubes. The stock solution can be stored at -80°C for a long term.

### 7. Cell cytopreserving solution

The specific formula of cell cytopreserving solution is shown in Table 15.

**Table 15 Cell Cytopreserving Solution**

| Reagent | | Brand Article No. | Dosage |
|---|---|---|---|
| Advanced medium | DMEM/F12 culture | Gibco#12634010 | 20 mL |
| DMSO | | Sigma#D2438 | 2 mL |
| 1% methylcellulose solution | | | 1 mL |

The cell cytopreserving solution needs to be prepared just before use.

In Table 15, the preparation of 1% methylcellulose solution is shown in Table 16.

**Table 16 1% Methylcellulose Solution (10 mL)**

| Reagent | Brand Article No. | Dosage | Final Concentration |
|---|---|---|---|
| Methylcellulose Ultrapure water | Sigma#M7027 | 0.1 g Added to 10 mL | 10g/L |

The prepared 1% methylcellulose solution can be stored at 4°C for a long time.

### Embodiment 2. Obtaining a postoperative specimen of a solid tumor tissue of lung cancer

1. The inventor cooperated with national grade-3, first-class hospitals (in China), and the cooperation passed the formal medical ethical review.
2. The attending physician selected the enrolled patients according to the clinical indications specified in the medical guidelines, and selected suitable samples for in vitro culture according to the intraoperative clinical indications; the selection criteria of samples were as follows: samples of primary lung cancer with II or III pathological stage and pathological types of non-small-cell lung cancer or small cell lung cancer, weighing more than 20 mg.
3. The attending physician provided a patient's basic clinical information, such as gender, age, medical history, family history, smoking history, pathological stages and types, clinical diagnosis, etc. The information related to patient privacy, such as patient's name and ID number, was concealed and replaced by a unified experimental number, and the naming principle of the experimental number was the 8-digit date of sample collection + last four digits of the patient's admission number. For example, for a sample provided on January 1, 2018, if the patient's admission number was T001512765, the sample experiment number was 201801012765.
4. During the operation, a surgeon collected a fresh specimen in a sterile environment of an operating room and placed it in a sample preservation solution prepared in advance (see Embodiment 1). The sample was temporarily stored on ice after ex vivo and transported to a laboratory within two hours for further operations.

### Embodiment 3. Performing predissociation treatment for a solid tumor tissue sample of lung cancer

The following steps were operated on ice, and the whole operation process was completed within 10 minutes.

All the surgical apparatuses used in the following embodiments were used only after high temperature and high-pressure sterilization and oven drying in advance.
1. The sample was weighed.
2. The sample surface was washed with 75% (percentage by volume) ethanol for 10 to 30 seconds.
3. The sample was washed with a sample washing solution for 5 times and washed with a sterile PBS solution for 5 times.
4. Adipose tissues, connective tissues and necrotic tissues in the sample were carefully peeled off with ophthalmic scissors, ophthalmic forceps, scalpel and other apparatuses.

### Embodiment 4. Dissociating the solid tumor tissue sample of lung cancer

All the surgical apparatuses used in the following embodiments can only be used after high temperature and high-pressure sterilization and oven drying in advance.
1. The tissues were cut into small pieces of about 1mm³ with ophthalmic scissors.
2. The tissue sample cut into pieces was treated with a sample dissociation solution preheated to 37°C in advance according to the dosage of 0.1 mL of sample dissociation solution (Table 3) per 1 mg of tissue, and the sample was dissociated at 37 °C for 15 minutes to 3 hours. The dissociation of the sample was observed under a microscope every 15 minutes until a large number of individual cells were observed.
3. The dissociation reaction was terminated with a 10× volume of digestion termination solution (Table 7), and the cell suspension was collected.
4. The cell suspension was filtered with a 100 µm sterile cell strainer to remove tissue debris and adherent cells.
5. The cell suspension was centrifugated at 800 g at room temperature for 10 minutes, and the supernatant was discarded.
6. The cells were resuspended with 5 mL of sterile PBS and centrifugated at 800 g at room temperature for 10 minutes, and the supernatant was discarded.
7. The cell precipitation was resuspended with the culture medium for primary cells from solid tumor tissues of lung cancer (Table 8), the cell state was observed under a microscope, and the cells were counted.

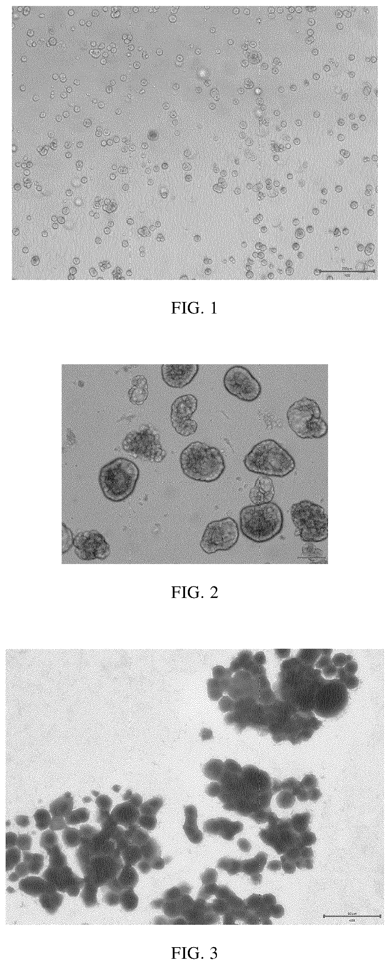

In addition to tumor cells, there were also a large number of various types of other cells, such as red blood cells, lymphocytes and fiber cells, which were mixed in the single cell suspension obtained by dissociation, as shown in FIG. 1. One of the advantages of the present method is that only the cancer cells could be amplified in the subsequent culture process, the proportion of other cells gradually decreases or even disappears, and primary tumor cells of lung cancer with a high purity are finally obtained.

### Embodiment 5. Culturing primary cells from solid tumor of lung cancer

1. A low-attachment-surface was used for suspension-culturing primary cells from solid tumor tissues of lung cancer, and the culture medium in use was the culture medium for primary cells from solid tumor tissues of lung cancer in example 1; taking a 6-well plate as an example, and cells were planked at a density of 10⁶ cells per well in a cell incubator under the condition of 37°C and 5% CO₂.
2. The cell state was observed every day, and the culture medium was replaced every 3 days until the cell masses with a diameter of about 100 µm were formed.

As shown in FIG. 2, after 3-10 days of culturing, cancer cells were amplified, cell masses with a diameter of 100 µm were formed, the total number of tumor cells exceeded 10⁷, and other types of cells significantly reduced or even disappeared. After a large number of sample tests, the success rate of the present method for culturing in vitro primary tumor cells from solid tumor of lung cancer could reach 70%.

### Embodiment 6. Passaging primary cells from solid tumor tissues of lung cancer

1. The cell masses in a culture dish were collected and centrifugated at 800 g at room temperature for 10 minutes, and the supernatant was discarded.
2. The cell masses were washed with sterile PBS solution and centrifugated at 800 g at room temperature for 10 minutes, and the supernatant was discarded.
3. The cell masses were resuspended with a cell digestion solution (Table 6) and dissociated at 37°C. The dissociation of the cell masses was observed under the microscope every 5 minutes until all the cell masses were dissociated into individual cells.
4. The dissociation reaction was terminated with a 10× volume of digestion termination solution (Table 7), and the cell suspension was collected.
5. The cell suspension was centrifugated at 800 g at room temperature for 10 minutes, and the supernatant was discarded.
6. The cell precipitation was resuspended with the culture medium for primary cells from solid tumor tissues of lung cancer (Table 8), and the cells were counted.
7. A low-attachment-surface was used for culturing primary cells from solid tumor of lung cancer, and the culture medium in use was the culture medium for primary cells from solid tumor of lung cancer in example 1; a 6-well plate was taken as an example, and cells were planked at a density of 10⁶ cells per well in a cell incubator under the condition of 37°C and 5% CO₂.

### Embodiment 7. Cytopreserving primary cells from solid tumor of lung cancer

The suspension-cultured primary cells from solid tumor of lung cancer by could be cytopreserved after 2-3 passages and amplifications:
1. The cell masses in a culture dish were collected and centrifugated at 800 g at room temperature for 10 minutes, and the supernatant was discarded.
2. The cell masses were washed with sterile PBS solution and centrifugated at 800 g at room temperature for 10 minutes, and the supernatant was discarded.
3. The cell masses were resuspended with a cell digestion solution (Table 6) and digested at 37°C. The digestion of the cell masses was observed under the microscope every 15 minutes until all the cell masses were digested into individual cells.
4. The digestion reaction was terminated with a 10× volume of digestion termination solution (Table 7), the cell suspension was collected, and the cells were counted.
5. The cell suspension was centrifugated at 800 g at room temperature for 10 minutes, and the supernatant was discarded.
6. The cell precipitation was resuspended at a density of 10⁶/mL with cell cytopreserving solution (Table 15), each 2 mL cytopreserving tube contained 1 mL of cell suspension, and the cell suspension was cytopreserved overnight in a gradient cooling box and transferred into liquid nitrogen for a long-term preservation.

### Embodiment 8. Resuscitating primary cells from solid tumor of lung cancer

Primary cells from solid tumor of lung cancer preserved in liquid nitrogen were resuscitated:
1. 37°C sterile water was prepared five minutes in advance.
2. The cytopreserving tubes were removed from liquid nitrogen, and the cells were quickly melted in 37°C sterile water.
3. The cell suspension was centrifugated at 800 g at room temperature for 10 minutes, and the supernatant was discarded.
4. The cell precipitation was resuspended with the culture medium for primary cells from solid tumor tissues of lung cancer (Table 8), the primary cells from solid tumor of lung cancer were cultured using a low-attachment-surface, the cells in each tube were resuscitated into a 3.5 cm culture dish, and the cells were cultured in a cell culturing incubator under the condition of 37 °C and 5% CO₂.

### Embodiment 9. HE staining identification of primary cells from solid tumor of lung cancer

Description of reagent consumables to be used in the following embodiment:
HE Staining Kit (Beijing Solarbio Science & Technology Co., Ltd., #G1120);
Cationic anticreep slide (ZSGB-BIO);
Xylene, methanol, acetone (Chemical Reagent Beijing Co., Ltd., analytical pure);
Neutral resin adhesive (Beijing Yili Fine Chemicals Co., Ltd.).
   1. Suspended primary cells from solid tumor of lung cancer obtained by culturing with the culture medium for primary cells from solid tumor tissues of lung cancer (Table 8, wherein the final concentration of human recombinant protein EGF was 50 ng/mL; the final concentration of human recombinant protein bFGF was 20 ng/mL; the final concentration of human recombinant protein MSG was 20 ng/mL; the final concentration of cortisol was 20 ng/mL; and the final concentration of Y-27632 was 10 µM) were prepared into a cell suspension with a concentration of 10⁴/mL, and 10 µL of the cell suspension was added dropwise on a cationic anti-creep slide to be dried in the air.
   2. 50 µL of a methanol/acetone mixture (volume ratio 1:1) pre-cooled at 4 °C was carefully added dropwise on the air-dried cells, and then the slide was placed in a refrigerator at 4 °C for fixing for 10 minutes.
   3. The slide for fixing the cells was taken out and naturally dried at room temperature.
   4. The slide was washed twice with 200 µL of PBS.
   5. 100 µL of hematoxylin staining solution was added for staining for 1 minute when the moisture on the slide was slightly dried.
   6. The hematoxylin staining solution was sucked up, and the slide was washed for 3 times with 200 µL of tap water.
   7. 100 µL of differentiation solution was added dropwise for differentiation for 1 minute.
   8. The differentiation solution was sucked up, and the slide was washed twice with tap water and washed with distilled water once.
   9. The moisture on the slide surface was sucked up, and 200 µL of eosin stain was added dropwise for staining for 40 s.
   10. The eosin stain was sucked up, and rinsing and dehydration were performed successively with 75%, 80%, 90% and 100% ethanol for 20 s, 20 s, 40 s and 40 s.
   11. After the ethanol was dried in the air, 50 µL of dimethylbenzene was added dropwise for cell permeability.
   12. After dimethylbenzene was completely dried, a drop of neutral resin adhesive was added, and the slide was sealed with a cover glass, observed under the microscope and photographed.

FIG. 3 shows a HE staining effect image of primary tumor cells from solid tumor of lung cancer obtained by in vitro culture. It can be seen that these cells generally have the characteristics of cancer cells, such as high nuclear cytoplasmic ratio, hyperchromasia, chromatin condensation in nucleus, multinucleation, uneven cell size, etc.

### Embodiment 10. Immunofluorescence staining identification of primary cells from solid tumor of lung cancer

Description of reagents to be used in the following embodiments:
Paraformaldehyde (Chemical Reagent Beijing Co., Ltd., analytical pure), paraformaldehyde powder was dissolved with ultrapure water to form 4% (4 g/100 mL) paraformaldehyde solution;
Methanol and dimethyl sulfoxide (Chemical Reagent Beijing Co., Ltd., analytical pure);
Hydrogen peroxide (Chemical Reagent Beijing Co., Ltd., 35%);
Methanol, dimethyl sulfoxide and 35% hydrogen peroxide were mixed in the ratio of 4:4:1 (volume ratio) to form Dan's rinsing solution;
Bovine serum albumin (Sigma, #A1933), bovine serum albumin was dissolved with PBS solution to form 3% (3 g/100 mL) BSA solution;
Immunofluorescence primary antibody (Abcam, #ab17139);
Immunofluorescence secondary antibody (CST, #4408);
Hoechst staining solution (Beijing Solarbio Science & Technology Co., Ltd., #C0021);
The masses of primary cells from solid tumor of lung cancer obtained by culturing with the culture medium for primary cells from solid tumor of lung cancer (Table 8, wherein the final concentration of human recombinant protein EGF was 50 ng/mL; the final concentration of human recombinant protein bFGF was 20 ng/mL; the final concentration of human recombinant protein MSP was 20 ng/mL; the final concentration of cortisol was 20 ng/mL; and the final concentration of Y-27632 was 10 µM) were subject to immunofluorescence staining according to the following steps, the primary antibody was CK8+CK18, and epithelial-derived cells were characterized.
   1. The masses of primary cells from solid tumor tissues of lung cancer in the culture dish were collected and washed once with PBS, and then the cell precipitation was resuspended with 4% paraformaldehyde and fixed at 4 °C overnight.
   2. The cell solution was centrifugated at 800 g, the supernatant was discarded, and the cell precipitation was resuspended with a precooled methanol solution and placed on ice for 1 hour.
   3. The cell solution was centrifugated at 800 g, the supernatant was discarded, and the cell precipitation was resuspended with Dan's rinsing solution and placed at room temperature for 2 hours.
   4. The cell solution was centrifugated at 800 g, the supernatant was discarded, and the cells were washed with 75%, 50% and 25% (percentage by volume) methanol solution diluted with PBS, 10 minutes each time.
   5. The cell solution was centrifugated at 800 g, the supernatant was discarded, and the cell precipitation was suspended with a 3% BSA solution and sealed at room temperature for 2 hours.
   6. The primary antibody was diluted with a 3% BSA solution at a ratio of 1:500, the cell precipitation was resuspended with the antibody diluent (3% BSA solution), and the primary antibody was placed at 4 °C overnight.
   7. The cell solution was centrifugated at 800 g, the supernatant was discarded, and the cell precipitation was washed for 5 times with PBS solution, with 20 minutes each time.
   8. The secondary antibody was diluted with a 3% BSA solution at a ratio of 1:2,000, the cell precipitation was resuspended with the antibody diluent (3% BSA solution), and the secondary antibody was placed at room temperature for 2 hours.
   9. The cell solution was centrifugated at 800 g, the supernatant was discarded, and the cell precipitation was washed for 5 times with PBS solution for 20 minutes each time.
   10. 100× Hoechst staining solution was added at a volume ratio of 1/100 for staining at room temperature for 20 minutes.
   11. The cell precipitation was washed twice with PBS solution, for 10 minutes each time. The staining of the cell masses was observed using a laser confocal microscope.

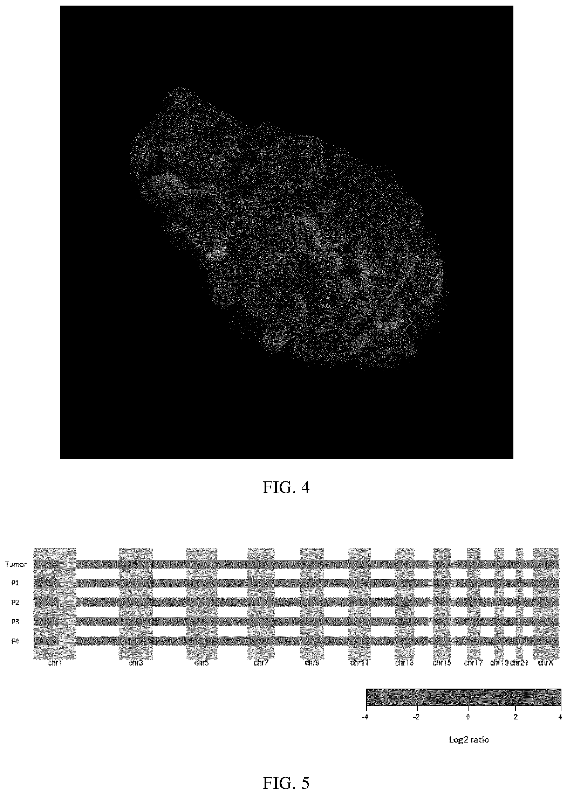

FIG. 4 shows an immunofluorescence staining effect image of primary cell masses from solid tumor of lung cancer cultured in vitro. It can be seen that all the cells constituting the cell masses are CK8/CK18 positive and epithelial-derived, which confirms that the tumor cells obtained by the present method are those with a high purity. Immunofluorescence staining identification was performed on 20 primary cultures of solid tumor samples of lung cancer, and the statistical results showed that the proportion of tumor cells in the primary cells from solid tumor of lung cancer obtained in the present method reached 75%-95% (Table 17).

**Table 17 Immunofluorescence Staining Identification of Primary Cultures of Lung Cancer Samples**

| S/N | Sample No. | Positive Rate of CK8/CK18 |
|---|---|---|
| 1 | 20160914ZDH0085 | 87.2% |
| 2 | 20160921SMY3832 | 89.1% |
| 3 | 20160921LXH3743 | 93.0% |
| 4 | 20160922XGX3936 | 82.5% |
| 5 | 20160928CLP9564 | 77.9% |
| 6 | 20161026XTT6317 | 91.4% |
| 7 | 20161108JSM7314 | 88.8% |
| 8 | 20161110ZZL3204 | 79.6% |
| 9 | 20161110WJj7710 | 82.5% |
| 10 | 20161114NSP7802 | 93.8% |
| 11 | 20161114YSX7805 | 89.5% |
| 12 | 20161115MZL4461 | 86.8% |
| 13 | 20161123GPF8479 | 92.7% |
| 14 | 20161201WPF9236 | 77.4% |
| 15 | 20161201WBY9242 | 84.0% |
| 16 | 20161206WYQ9298 | 93.5% |
| 17 | 20161223ZGL3074 | 91.2% |
| 18 | 20161226CSH1078 | 89.3% |
| 19 | 20170111YXM6263 | 81.2% |
| 20 | 20170216LJR1120 | 84.3% |

### Embodiment 11. Primary cell cultures and primary tumor tissues from solid tumor tissues of lung cancer

The DNA extraction mentioned in the following embodiment was performed with TIANGEN blood/tissue/cell genome Extraction Kit (DP304).

The library building mentioned in the following embodiment was performed with NEB DNA Sequencing and Library Building Kit (E7645).

The high-throughput sequencing mentioned in the following embodiment refers to the Illumina HiSeq X-ten sequencing platform.
1. A solid tumor sample of lung cancer was obtained, 10 mg of solid tumor sample of lung cancer was firstly taken for DNA extraction, library building and whole-genome high-throughput sequencing (WGS), and with a sequencing depth of 30× before in vitro culture operations were performed, the remaining solid tumor samples were used for culturing in vitro primary cells from solid tumor of lung cancer (the specific method refers to that in the previously described embodiment).
2. Lung cancer tissues were treated and cultured with the culture medium for primary cells from solid tumor of lung cancer (Table 8, wherein the final concentration of human recombinant protein EGF was 50 ng/mL; the final concentration of human recombinant protein bFGF was 20 ng/mL; the final concentration of human recombinant protein MSP was 20 ng/mL; the final concentration of cortisol was 20 ng/mL; and the final concentration of Y-27632 was 10 µM) for a period of time to form cell masses with a diameter of more than 100 µm, which were recorded as P0-generation cells, followed by P1, P2, ..., Pn successively according to the number of passages. 10⁶ cells were taken respectively from P1, P2, P3 and P4-generation primary cell cultures from solid tumor of lung cancer for DNA extraction, library building and whole-genome high-throughput sequencing (WGS), with a sequencing depth of 30×.
3. Copy number variation analysis (CNV) was performed based on the sequencing results of each group, and the copy number variations between primary tumor tissues of lung cancer and all generations of primary cell cultures from solid tumor of lung cancer were compared. As shown in FIG. 5, the copy number variation of all generations of primary cell cultures from solid tumor of lung cancer (P1, P2, P3 and P4) was highly consistent with that of the primary tumor tissue (Tumor) of lung cancer, so the primary cells from solid tumor of lung cancer obtained by the present method could represent the real condition of the patient's primary tumor.

### Embodiment 12. Comparing the success rates of different primary cell culture media for culturing primary cells from solid tumor of lung cancer

The operation methods and processes for culturing primary cells from all samples in this embodiment were identical (as mentioned above), and the only difference lied in the culture medium formula. Various tested primary cell culture media were shown in Table 18. Wherein Scheme D is the formula used in the present invention, with details in Table 8 (wherein the final concentration of human recombinant protein EGF was 40 ng/mL; the final concentration of human recombinant protein bFGF was 20 ng/mL; the final concentration of human recombinant protein MSP was 20 ng/mL; the final concentration of cortisol was 20 ng/mL; and the final concentration of Y-27632 was 5 µM)

**Table 18 Primary Cell Culture Medium Formula For Test (100 mL)**

| Culture Medium | Reagent | Brand Article No. | Dosage | Final Concentration |
|---|---|---|---|---|
| Scheme A | mTeSR^{™}1 medium | Stemcell#05850 | 100 mL | |
| Scheme B | Alveolar Epithelial Cell Medium | ScienCell#3201 | 100 mL | |
| Scheme C | Dual-antibody P/S | Gibco#15140122 | 1 mL | 1% |
| | HEPES | Gibco#15630080 | 1 mL | 10 mM |
| | 1,000× human recombinant protein | 1,000× stock solution | 250 µL | 50 ng/mL |
| | EGF | | | |
| | 1,000× human recombinant protein bFGF | 1,000× stock solution | 100 µL | 20 ng/mL |
| | DMEM/F12 culture medium | Gibco# 14170161 | Added to 100 mL | |
| Scheme D | See Table 8 | | | |

After being prepared, the primary cell culture media were filtered and sterilized with a 0.22 µM syringe-driven strainer (Millipore SLGP033RS). The primary cell culture media can be stored at 4 °C for two weeks.

20 solid tumor tissue samples of lung cancer were treated according to each of the four primary cell culture medium schemes, the sample treatment and culture operations were performed according to the methods described in embodiments 3, 4 and 5, and the success rates of those primary cell culture media for culturing primary cells from solid tumor tissues of lung cancer were counted after 10 days of culturing, as shown in Table 19:

**Table 19 Culturing in Different Media**

| Sample Preservation Solution Scheme | Success rate |
|---|---|
| Scheme A | 0% (0/20) |
| Scheme B | 25% (5/20) |
| Scheme C | 25% (5/20) |
| Scheme D | 75% (15/20) |

It can be seen that primary cell culture media have an extremely great impact on the success rate of culturing primary cells from solid tumor cells of lung cancer, and the culture medium for primary cells from solid tumor tissues of lung cancer used in the present invention (Table 8) can, to the greatest extent, stimulate the proliferation of cancer cells in the solid tumor tissue sample of lung cancer and improve the success rate of culturing primary cells from solid tumor of lung cancer.

### Embodiment 13. Comparing success rates of different sample preservation solutions for culturing primary cells from solid tumor of lung cancer

The operation methods and processes for culturing primary cells from all samples in this embodiment were identical (as mentioned above), and the only difference lied in the sample preservation solution formula. Various tested sample preservation solutions are shown in Table 20. Wherein Scheme E is the formula used in the present invention, and the details are shown in Table 1.

**Table 20 Formula of Sample Preservation Solution for Test (100 mL)**

| Sample Preservation Solution Scheme | Reagent | Brand Article No. | Dosage | Final Concentration |
|---|---|---|---|---|
| Scheme A | HBSS | Gibco# 14170161 | 100 mL | |
| Scheme B | Dual-antibody P/S | Gibco#15140122 | 1 mL | 1% |
| | HBSS | Gibco# 14170161 | Added | |
| | | | to 100 mL | |
| Scheme C | Dual-antibody P/S | Gibco#15140122 | 1 mL | 1% |
| | DMEM culture medium | Gibco#11965-092 | Added to 100 mL | |
| Scheme D | Dual-antibody P/S | Gibco#15140122 | 1 mL | 1% |
| | Fetal bovine serum | Gibco#16000-044 | 10 mL | 10% |
| | DMEM culture medium | Gibco#11965-092 | Added to 100 mL | |
| Scheme E | See Table 1 | | | |

After being prepared, those sample preservation solutions in the above table were sub-packaged in 15 mL centrifuge tubes, and each tube was filled with 5 mL of sample preservation solutions. The sample preservation solution sub-packaged can be stored at 4°C for 1 month.

20 solid tumor tissue samples of lung cancer were treated according to each of the five sample preservation solution schemes, and the samples were detached and temporarily preserved at 4 °C in the sample preservation solution; the sample treatment and culture operations were performed according to the methods described in embodiments 3, 4 and 5 two hours after detachment, and the success rates of those sample preservation solutions for culturing primary cells from solid tumor of lung were counted after 10 days of culturing, as shown in Table 21:

**Table 21 Culturing in Different Sample Preservation Solutions**

| Sample Preservation Solution Scheme | Success rate | Bacteria/Fungus contamination rate |
|---|---|---|
| Scheme A | 40% (8/20) | 15% (3/20) |
| Scheme B | 55% (11/20) | 0% (0/20) |
| Scheme C | 55% (11/20) | 0% (0/20) |
| Scheme D | 70% (14/20) | 0% (0/20) |
| Scheme E | 75% (15/20) | 0% (0/20) |

It can be seen that different sample preservation solutions have a great impact on the culture success rate of culturing primary cells from solid tumor of lung cancer, and the sample preservation solution used in the present invention (Table 1) can, to the greatest extent, protect the activity of cancer cells in the solid tumor tissue sample of lung cancer and improve the success rate.

### Embodiment 14. Comparing success rates of different sample dissociation solutions for culturing primary cells from solid tumor tissues of lung cancer

The operation methods and processes of primary culturing of all samples in this embodiment were identical (as mentioned above), and the only difference lied in the sample dissociation solution formula. Various tested sample dissociation solutions are shown in Table 22. Wherein Scheme D is the formula used in the present invention, and the details are shown in Table 3.

**Table 22 Sample Dissociation Solution Formula for Test (10 mL)**

| Sample Dissociation Solution | Reagent | Brand Article No. | Dosage | Final Concentration |
|---|---|---|---|---|
| Scheme A | Trypsin | Gibco# 252,00056 | 10 mL | |
| Scheme B | 0.5M EDTA | Invitrogen#AM9261 | 10 µL | 5 mM |
| | DNAse | Thermo# EN0521 | 62.5 µL | 62.5 U/mL |
| | TrypLE^{™} Express | Gibco#12604013 | Added to 10 mL | |
| Scheme C | 10× collagenase IV | 10× stock solution | 1 mL | 200 U/mL |
| | DNAse | Thermo# EN0521 | 62.5 µL | 62.5 U/mL |
| | PBS | Gibco#21-040-CVR | Added to 10 mL | |
| Scheme D | See Table 3 | | | |

The sample dissociation solution needs to be prepared just before use.

20 samples of solid tumor tissue masses of lung cancer weighing more than 100 mg were selected and sub-packaged into four parts equally, and sample treatment and culture operations were performed with the above four sample dissociation solutions respectively according to the methods described in embodiments 3, 4 and 5. The success rates of sample dissociation solutions for culturing primary cells from solid tumor tissues of lung cancer were counted after 10 days of culturing, as shown in Table 23 below:

**Table 23 Culturing in Different Sample Dissociation Solutions**

| S/N | Sample No. | Scheme A | Scheme B | Scheme C | Scheme D |
|---|---|---|---|---|---|
| 1 | 20160822ZGQ2244 | Failed | Failed | Failed | Passed |
| 2 | 20160823CZH7746 | Failed | Failed | Failed | Failed |
| 3 | 20160823CXL5741 | Failed | Failed | Passed | Passed |
| 4 | 20160826LBL2723 | Failed | Passed | Failed | Passed |
| 5 | 20160901YZM2848 | Failed | Failed | Passed | Passed |
| 6 | 20160901FYR8262 | Failed | Failed | Failed | Failed |
| 7 | 20160902YXL8520 | Failed | Failed | Failed | Failed |
| 8 | 20160902ZGB3405 | Failed | Passed | Passed | Passed |
| 9 | 20160906YXY9476 | Failed | Passed | Passed | Passed |
| 10 | 20160919HQH3874 | Failed | Failed | Passed | Passed |
| 11 | 20161009FJX0000 | Failed | Failed | Failed | Failed |
| 12 | 20161009LFZ0000 | Failed | Failed | Failed | Passed |
| 13 | 20161010ZLH5257 | Failed | Passed | Passed | Passed |
| 14 | 20161011GLZ8283 | Failed | Failed | Failed | Failed |
| 15 | 20161026YYH6569 | Failed | Failed | Failed | Failed |
| 16 | 20161026ESP0641 | Failed | Failed | Failed | Passed |
| 17 | 20161027MGL7286 | Failed | Passed | Passed | Passed |
| 18 | 20161107WHP9893 | Failed | Failed | Passed | Passed |
| 19 | 20161111YZM2848 | Failed | Failed | Failed | Passed |
| 20 | 20170209WYT2997 | Failed | Failed | Passed | Passed |
| | Summary | 0% (0/20) | 25% (5/20) | 45% (9/20) | 70% (14/20) |

It can be seen that different sample dissociation solutions have a very great impact on the success rate of culturing primary cells from solid tumor tissues of lung cancer, and the sample dissociation solution used in the present invention (Table 3) can, to the greatest extent, isolate the cancer cells from solid tumor tissues of lung cancer and improve the success rate of culturing primary cells from solid tumor tissues of lung cancer.

### Embodiment 15. Comparing success rates of different cell digestion solutions for passaging primary cells from solid tumor tissues of lung cancer

The operation methods and processes for passaging primary cells of all samples in this embodiment were identical (as mentioned above), and the only difference lied in the cell digestion solution formula. Various tested cell digestion solution are shown in Table 24. Wherein Scheme D is the formula used in the present invention, and the details are shown in Table 6.

**Table 24 Cell Digestion Solution Formula for Test (10 mL)**

| Cell Digestion Solution | Reagent | Brand Article No. | Dosage | Final Concentration |
|---|---|---|---|---|
| Scheme A | Trypsin | Gibco# 252,00056 | 10 mL | |
| Scheme B | 0.5M EDTA | Invitrogen#AM9261 | 10 µL | 5 mM |
| | TrypLE^{™} Express | Gibco#12604013 | Added to 10 mL | |
| Scheme C | 10× collagenase I | 10× stock solution | 1 mL | 200 U/mL |
| | 10× collagenase | 10× stock solution | 1 mL | 200 U/mL |
| | IV PBS | Gibco#21-040-CVR | Added to 10 mL | |
| Scheme D | See Table 6 | | | |

The cell digestion solution needs to be prepared just before use.

20 successfully cultured solid tumor tissue samples of lung cancer were selected, and the primary cells from solid tumor of lung cancer obtained by culture were continuously passaged with the above four cell digestion solutions respectively according to the method described in Embodiment 6. Passaging (for no more than 10 times) was performed every time cancer cells amplified and formed cell masses with a diameter of 100 µm, and the maximum passaging number was recorded. The statistical results are as shown in Table 25:

**Table 25 Culturing in Different Cell Digestion Solutions**

| S/N | Sample No. | Scheme A | Scheme B | Scheme C | Scheme D |
|---|---|---|---|---|---|
| 1 | 20160822ZGQ2244 | 1 | 3 | 5 | 9 |
| 2 | 20160823CXL5741 | 1 | 5 | 5 | 10 |
| 3 | 20160901YZM2848 | 1 | 3 | 4 | 10 |
| 4 | 20160902ZGB3405 | 1 | 3 | 5 | 9 |
| 5 | 20160906YXY9476 | 1 | 4 | 4 | 10 |
| 6 | 20160919HQH3874 | 2 | 6 | 7 | 10 |
| 7 | 20161009LFZ0000 | 1 | 2 | 3 | 8 |
| 8 | 20161010ZLH5257 | 1 | 3 | 4 | 8 |
| 9 | 20161026ESP0641 | 1 | 3 | 4 | 8 |
| 10 | 20161027MGL7286 | 1 | 5 | 6 | 10 |
| 11 | 20161107WHP9893 | 1 | 2 | 4 | 9 |
| 12 | 20161110ZZL3204 | 2 | 4 | 6 | 10 |
| 13 | 20161110WJj7710 | 1 | 5 | 5 | 10 |
| 14 | 20161111YZM2848 | 1 | 4 | 5 | 10 |
| 15 | 20170111YXM6263 | 1 | 4 | 6 | 10 |
| 16 | 20161114NSP7802 | 1 | 2 | 3 | 8 |
| 17 | 20161114YSX7805 | 1 | 5 | 5 | 9 |
| 18 | 20170209WYT2997 | 1 | 4 | 5 | 8 |
| 19 | 20170216LJR1120 | 1 | 3 | 3 | 8 |
| 20 | 20170417LFX7932 | 1 | 3 | 4 | 9 |
| | Summary of passaging number | 1-2 times | 2-6 times | 3-7 times | More than 8 times |

It can be seen that different cell digestion solutions have a very great impact on the success rate of passaging primary cells from solid tumor of lung cancer, and the cell digestion solution used in the present invention (Table 6) can mildly dissociate the cancer cells in the cell masses to continuously passage the samples and maintain the activity of primary cells from solid tumor of lung cancer.

### Embodiment 16. Culturing primary tumor cells of lung cancer from puncture microsamples

The operation methods and processes of primary culturing of all samples in this embodiment were identical with the methods for culturing primary cells from solid tumor of lung cancer (as mentioned above), and the only difference lied in the sample source which was unified as two puncture samples (Table 26).

**Table 26 Culturing of Primary Cells from Puncture Samples of Lung Cancer**

| S/N | Sample No. | Culture |
|---|---|---|
| 1 | 20190107YJD0928 | Passed |
| 2 | 20190113KFH8364 | Failed |
| 3 | 20190114PSY7482 | Passed |
| 4 | 20190114NCC4701 | Passed |
| 5 | 20190117LCN3957 | Passed |
| 6 | 20190125GCY3572 | Passed |
| 7 | 20190201HZY0486 | Passed |
| 8 | 20190201GDN4967 | Failed |
| 9 | 20190205DHB0029 | Passed |
| 10 | 20190207DJB9586 | Failed |
| Success Rate | | 7/10 |

It can be seen from Table 26 that the microsamples obtained by puncture can still culture primary cells of lung cancer at a higher success rate.

### Embodiment 17. Preparing a reagent for culturing primary cells from pleural effusion of lung cancer

### 1. Cell Isolation Buffer (100 mL)

The specific formula of cell isolation buffer (100 mL) is shown in Table 27:

**Table 27 Cell Isolation Buffer (100 mL)**

| Reagent | Brand Article No. | Dosage | Final Concentration |
|---|---|---|---|
| Dual-antibody P/S | Gibco#15140122 | 1 mL | 1% |
| 1,000× heparin sodium solution | 1,000× stock solution | 0.1 mL | 10 IU/mL |
| PBS | Gibco#21-040-CVR | Added to 100 mL | |

The prepared cell isolation buffer can be stored at 4 °C for 1 month.

In Table 27, the preparation of the heparin sodium solution is shown in Table 28.

**Table 28 1,000× Heparin Sodium (1 mL)**

| Reagent | Brand Article No. | Dosage | Final Concentration |
|---|---|---|---|
| Heparin sodium Ultrapure water | Solarbio#H8270 | 10 kIU Added to 1 mL | 10 kIU/mL |

1,000× heparin sodium solution needs to be prepared just before use.

### 2. Cell Digestion Solution (10 mL)

The specific formula of cell digestion solution (10 mL) is shown in Table 6 (the same as the formula of the cell digestion solution used for culturing primary cells from solid tumor of lung cancer).

### 3. Digestion Termination solution (100 mL)

The specific formula of digestion termination solution (100 mL) is shown in Table 7 (the same as the formula of the digestion termination solution used for culturing primary cells from solid tumor of lung cancer).

### 4. Culture medium for primary tumor cells from pleural effusion of lung cancer (100 mL)

The specific formula of culture medium for primary cell from pleural effusion of lung cancer (100 mL) is shown in Table 8 (the same as the formula of the culture medium for primary tumor cells from solid tumor of lung cancer used for culturing primary cells from solid tumor tissues of lung cancer).

### 5. Cell cytopreserving solution

The specific formula of cell cytopreserving solution (10 mL) is shown in Table 15 (the same as the formula of the cell cytopreserving solution used for culturing primary cells from solid tumor of lung cancer).

### Embodiment 18. Obtaining pleural effusion samples of lung cancer

1. The inventor cooperated with national grade-3, first-class hospitals (in China), and the cooperation passed the formal medical ethical review.
2. The attending physician selected the enrolled patients according to the clinical indications specified in the medical guidelines, and selected suitable samples for in vitro culture according to the intraoperative clinical indications; the selection criteria of samples were as follows: primary lung cancer with IV pathological stage and pathological types of non-small-cell lung cancer or small cell lung cancer, and patients accompanied by malignant pleural effusion to be drained, with a drainage amount of not less than 50 mL.
3. The attending physician provided a patient's basic clinical information, such as gender, age, medical history, family history, smoking history, pathological stages and types, clinical diagnosis, etc. The information related to patient privacy, such as patient's name and ID number, was concealed and replaced by a unified experimental number, and the experimental number was equal to the 8-digit date of sample collection + last four digits of the patient's admission number. For example, for a sample provided on January 1, 2018, if the patient's admission number was T001512765, the sample experiment number was 201801012765.
4. Pleural effusion samples were collected by the attending physician using sterile apparatuses and containers. After being detached, the sample was temporarily preserved on ice and transported to a laboratory for the next operation within 72 hours.

### Embodiment 19. Pretreating pleural effusion samples of lung cancer

The following embodiment was operated on ice:
1. The pleural effusion sample of lung cancer was kept still on ice for about 30 minutes, so that blood clots and large insoluble solids in the sample were settled to the bottom of the sample tube.
2. The supernatant was carefully transferred into a 50 mL sterile centrifuge tube.
3. The cell suspension was centrifugated at 2,000 g at room temperature for 5 minutes, and the supernatant was discarded.
4. The cell precipitation was resuspended with a cell isolation buffer (Table 27) and centrifuged at 2,000 g for 5 minutes, and the supernatant was discarded.
5. The cell precipitation was resuspended with a cell isolation buffer (Table 27), and the cell concentration was adjusted to 10⁷-10⁸ cells/mL.

### Embodiment 20. Density gradient centrifugation of pleural effusion samples of lung cancer

1. Ficoll cell separation solution (MP#50494) of the same volume as the cell suspension was taken by a 50 mL sterile centrifuge tube.
2. The cell suspension was carefully added to the upper layer of the cell separation solution, so that a clear interface was formed between them.
3. The cell suspension was horizontally centrifuged at 2,000 g at room temperature for 20 minutes.
4. A white membrane in an intermediate layer was sucked into a new tube.
5. The cell precipitation was resuspended with 20 mL sterile PBS and centrifuged at 1,500 g at room temperature for 10 minutes, and the supernatant was discarded.
6. The cell precipitation was resuspended with the culture medium for primary cell from pleural effusion of lung cancer (Table 8), the cell state was observed under a microscope, and the cells were counted.

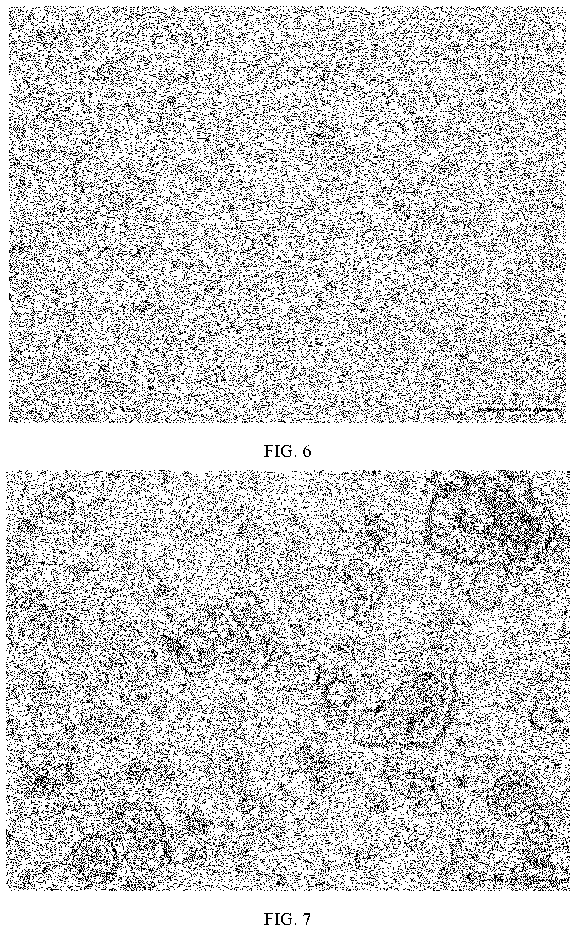

In addition to tumor cells, there were also a large number of various types of other cells, such as red blood cells, lymphocytes and fiber cells, mixed in the single cell suspension isolated from the pleural effusion samples of lung cancer, as shown in FIG. 6. One of the advantages of the present method is that only the cancer cells could be amplified in the subsequent culture process, the proportion of other cells gradually decreases or even disappears, and primary tumor cells of lung cancer with a high purity are finally obtained.

### Embodiment 21. Culturing primary tumor cells from pleural effusion of lung cancer

1. A low-attachment-surface was used for suspension-culturing primary tumor cells from pleural effusion of lung cancer; a 6-well plate was taken as an example, and cells were planked at a density of 10⁶ cells per well in a cell incubator under the condition of 37°C and 5% CO₂.
2. The cell state was observed every day, and the culture medium was replaced every 3 days until the cells formed masses with a diameter of about 100 µm.

As shown in FIG. 7, after 3-10 days of culturing, cancer cells were amplified, cell masses with a diameter of 100 µm were formed, the total number of tumor cells exceeded 10⁷, and other types of cells significantly reduced or even disappeared. After a large number of sample tests, the success rate of the present method for culturing in vitro primary tumor cells from pleural effusion of lung cancer could reach 80%.

### Embodiment 22. Passaging primary tumor cells from pleural effusion of lung cancer

1. The cell masses in a culture dish were collected and centrifugated at 800 g at room temperature for 10 minutes, and the supernatant was discarded.
2. The cell masses were washed with sterile PBS solution and centrifugated at 800 g at room temperature for 10 minutes, and the supernatant was discarded.
3. The cell masses were resuspended with a cell digestion solution (Table 6) and digested at 37°C. The digestion of the cell masses was observed under the microscope every 5 minutes until all the cell masses were digested into individual cells.
4. The dissociation reaction was terminated with a 10× volume of digestion termination solution (Table 7), and the cell suspension was collected.
5. The cell suspension was centrifugated at 800 g a room temperature for 10 minutes, and the supernatant was discarded.
6. The cell precipitation was resuspended with the culture medium for primary cells for pleural effusion of lung cancer (Table 8), and the cells were counted.
7. A low-attachment-surface was used for suspension-culturing primary cells of lung cancer; a 6-well plate was taken as an example, and cells were planked at a density of 10⁶ cells per well in a cell incubator under the condition of 37°C and 5% CO₂.

### Embodiment 23. Cytopreserving primary tumor cells from pleural effusion of lung cancer

The suspension-cultured primary tumor cells from pleural effusion of lung cancer could be cytopreserved after 2-3 passages and amplifications:
1. The cell masses in a culture dish were collected and centrifugated at 800 g at room temperature for 10 minutes, and the supernatant was discarded.
2. The cell masses were washed with sterile PBS solution and centrifugated at 800 g at room temperature for 10 minutes, and the supernatant was discarded.
3. The cell masses were resuspended with a cell digestion solution (Table 6) and digested at 37°C. The digestion of the cell masses was observed under the microscope every 5 minutes until all the cell masses were digested into individual cells.
4. The dissociation reaction was terminated with a 10× volume of digestion termination solution (Table 7), the cell suspension was collected, and the cells were counted.
5. The cell suspension was centrifugated at 800 g at room temperature for 10 minutes, and the supernatant was discarded.
6. The cell precipitation was resuspended at a density of 10⁶/mL with cell cytopreserving solution (Table 15), each 2 mL cytopreserving tube contained each 1 mL of cell suspension, and the cell suspension was cytopreserved overnight in a gradient cooling box and transferred into liquid nitrogen for a long-term preservation.

### Embodiment 24. Resuscitating primary tumor cells from pleural effusion of lung cancer

Primary tumor cells from pleural effusion of lung cancer preserved in liquid nitrogen could be resuscitated:
1. 37°C sterile water was prepared five minutes in advance.
2. The cytopreserving tubes were removed from liquid nitrogen, and the cells were quickly melted in 37°C sterile water.
3. The cell suspension was centrifugated at 800 g at room temperature for 10 minutes, and the supernatant was discarded.
4. The cell precipitation was resuspended with the culture medium for primary tumor cells from pleural effusion of lung cancer (Table 8), the primary tumor cells from pleural effusion of lung cancer were cultured using a low-attachment-surface, the cells in each tube were resuscitated into a 3.5 cm culture dish, and the cells were cultured in a cell Culturing incubator under the condition of 37 °C and 5% CO₂.

### Embodiment 25. HE staining identification of primary tumor cells from pleural effusion of lung cancer

Description of reagent consumables to be used in the following embodiment:
HE Staining Kit (Beijing Solarbio Science & Technology Co., Ltd., #G1120);
Cationic anticreep slide (ZSGB-BIO);
Xylene, methanol, acetone (Chemical Reagent Beijing Co., Ltd., analytical pure);
Neutral resin adhesive (Beijing Yili Fine Chemicals Co., Ltd.).
   1. Suspended primary tumor cells from pleural effusion of lung cancer obtained by culturing with the culture medium for primary cells from pleural effusion of lung cancer (Table 8, wherein the final concentration of human recombinant protein EGF was 60 ng/mL; the final concentration of human recombinant protein bFGF was 30 ng/mL; the final concentration of human recombinant protein MSG was 25 ng/mL; the final concentration of cortisol was 40 ng/mL; and the final concentration of Y-27632 was 15 µM) were prepared into a cell suspension with a concentration of 10⁴/mL, and 10 µL of the cell suspension was added dropwise on a cationic anti-creep slide to be dried in the air.
   2. 50 µL of a methanol/acetone mixture (volume ratio 1:1) pre-cooled at 4 °C was carefully added dropwise on the air-dried cells, and then the slide was placed in a refrigerator at 4 °C for fixing for 10 minutes.
   3. The slide for fixing the cells was taken out and naturally dried at room temperature.
   4. The slide was washed twice with 200 µL of PBS.
   5. 100 µL of hematoxylin staining solution was added for staining for 1 minute when the moisture on the slide was slightly dried.
   6. The hematoxylin staining solution was sucked up, and the slide was washed for 3 times with 200 µL of tap water.
   7. 100 µL of differentiation solution was added dropwise for differentiation for 1 minute.
   8. The differentiation solution was sucked up, and the slide was washed twice with tap water and washed with distilled water once.
   9. The moisture on the slide surface was sucked up, and 200 µL of eosin stain was added dropwise for staining for 40 s.
   10. The eosin stain was sucked up, and rinsing and dehydration were performed successively with 75%, 80%, 90% and 100% ethanol for 20 s, 20 s, 40 s and 40 s.
   11. After the ethanol was dried in the air, 50 µL of dimethylbenzene was added dropwise for cell permeability.
   12. After dimethylbenzene was completely dried, a drop of neutral resin adhesive was added, and the slide was sealed with a cover glass, observed under the microscope and photographed.

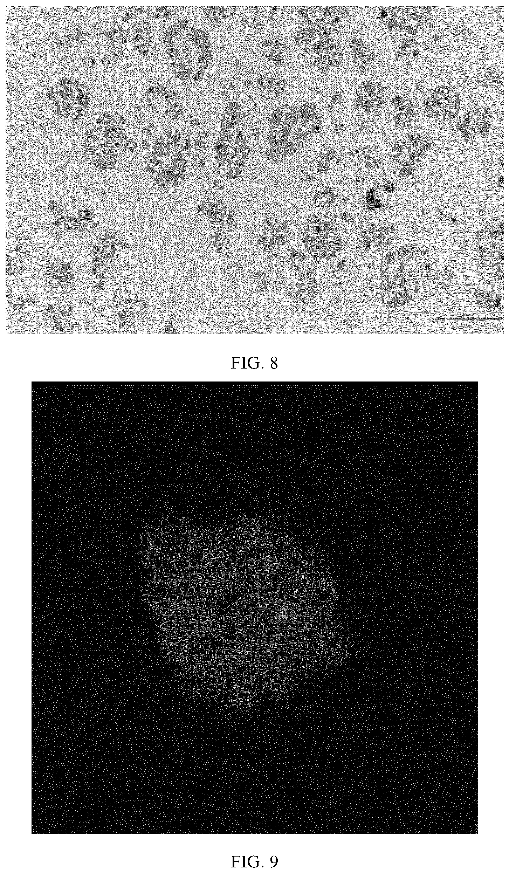

FIG. 8 shows an HE staining effect image of primary tumor cells from pleural effusion of lung cancer obtained by in vitro culture. It can be seen that these cells generally have the characteristics of cancer cells, such as high nuclear cytoplasmic ratio, hyperchromasia, chromatin condensation in nucleus, multinucleation, uneven cell size, etc.

### Embodiment 26. Immunohistochemical staining identification of primary tumor cells from pleural effusion of lung cancer

Description of reagents to be used in the following embodiment:
Paraformaldehyde (Chemical Reagent Beijing Co., Ltd., analytical pure), paraformaldehyde powder was dissolved with ultrapure water to form 4% paraformaldehyde solution;
Methanol and dimethyl sulfoxide (Chemical Reagent Beijing Co., Ltd., analytical pure);
Hydrogen peroxide (Chemical Reagent Beijing Co., Ltd., 35%);
Methanol, dimethyl sulfoxide and 35% hydrogen peroxide were mixed in the ratio of 4:4:1 to form Dan's rinsing solution;
Bovine serum albumin (Sigma, #A1933), bovine serum albumin was dissolved with PBS solution to form 3% BSA solution;
Immunofluorescence primary antibody (Abcam, #ab17139);
Immunofluorescence secondary antibody (CST, #4408);
Hoechst staining solution (Beijing Solarbio Science & Technology Co., Ltd., #C0021);
The masses of primary tumor cells from pleural effusion of lung cancer obtained by culturing with the culture medium for primary tumor cells from pleural effusion of lung cancer (Table 8, wherein the final concentration of human recombinant protein EGF was 60 ng/mL; the final concentration of human recombinant protein bFGF was 30 ng/mL; the final concentration of human recombinant protein MSP was 25 ng/mL; the final concentration of cortisol was 40 ng/mL; and the final concentration of Y-27632 was 15 µM) were subject to immunofluorescence staining according to the following steps, the primary antibody was CK8+CK18, and epithelial-derived cells were characterized.
   1. The masses of primary tumor cells from pleural effusion of lung cancer in the culture dish were collected and washed once with PBS, and then the cell precipitation was resuspended with 4% paraformaldehyde and fixed at 4 °C overnight.
   2. The cell solution was centrifugated at 800 g, the supernatant was discarded, and the cell precipitation was resuspended with a precooled methanol solution and placed on ice for 1 hour.
   3. The cell solution was centrifugated at 800 g, the supernatant was discarded, and the cell precipitation was resuspended with Dan's rinsing solution and placed at room temperature for 2 hours.
   4. The cell solution was centrifugated at 800 g, the supernatant was discarded, and the cells were washed with 75%, 50% and 25% methanol solution diluted with PBS, 10 minutes each time.
   5. The cell solution was centrifugated at 800 g, the supernatant was discarded, and the cell precipitation was suspended with a 3% solution and sealed at room temperature for 2 hours.
   6. The primary antibody was diluted with a 3% BSA solution at a ratio of 1:500, the cell precipitation was resuspended with the antibody diluent, and the primary antibody was placed at 4 °C overnight.
   7. The cell solution was centrifugated at 800 g, the supernatant was discarded, and the cell precipitation was washed for 5 times with PBS solution, with 20 minutes each time.
   8. The secondary antibody was diluted with a 3% BSA solution at a ratio of 1:2,000, the cell precipitation was resuspended with the antibody diluent, and the secondary antibody was placed at room temperature for 2 hours.
   9. The cell solution was centrifugated at 800 g, the supernatant was discarded, and the cell precipitation was washed for 5 times with PBS solution, with 20 minutes each time.
   10. 100× Hoechst staining solution was added at a volume ratio of 1/100 for staining at room temperature for 20 minutes.
   11. The cell precipitation was washed twice with PBS solution, with 10 minutes each time. The staining of the cell masses was observed using a laser confocal microscope.

FIG. 9 shows an immunofluorescence staining effect image of primary tumor cell masses from pleural effusion of lung cancer cultured in vitro. It can be seen that all the cells constituting the cell masses are CK8/CK18 positive and epithelial-derived, which confirms that the tumor cells obtained by the present method are those with a high purity. Immunofluorescence staining identification was performed based on 20 primary cultures of pleural effusion samples of lung cancer, and the statistical results showed that the proportion of tumor cells in the primary tumor cells from pleural effusion of lung cancer obtained in the present method reached 75%-95% (Table 29).

**Table 29 Immunofluorescence Staining Identification of Primary Cultures from Pleural Effusion of Lung Cancer**

| S/N | Sample No. | Positive Rate of CK8/CK18 |
|---|---|---|
| 1 | 20170623WJD4442 | 0.750587399 |
| 2 | 20171212WPS8666 | 0.931396528 |
| 3 | 20180104FZH0918 | 0.756076105 |
| 4 | 20180329LYD7566 | 0.933623143 |
| 5 | 20180404QX8612 | 0.909249588 |
| 6 | 20180408ZMY8630 | 0.876574815 |
| 7 | 20180419WCY8644 | 0.931840881 |
| 8 | 20180504YAQ1360 | 0.919277775 |
| 9 | 20180501WZM1432 | 0.759422386 |
| 10 | 20180518YJD2931 | 0.877793597 |
| 11 | 20180523WYG2813 | 0.921129568 |
| 12 | 20180523YBM3283 | 0.838792271 |
| 13 | 20180614QX8612 | 0.771108906 |
| 14 | 20180828JP1921 | 0.824987208 |
| 15 | 20180912LGL3201 | 0.922425562 |
| 16 | 20180709XP0862 | 0.840305017 |
| 17 | 20180711JDL7423 | 0.91463045 |
| 18 | 20180810LAJ9251 | 0.923325274 |
| 19 | 20180817ZRS0912 | 0.905725702 |
| 20 | 20180821XHB2551 | 0.935186153 |

### Embodiment 27. Primary tumor cell cultures and primary tumor tissues from pleural effusion of lung cancer

The DNA extraction mentioned in the following embodiment was performed with TIANGEN blood/tissue/cell genome Extraction Kit (DP304).

The library building mentioned in the following embodiment was performed with NEB DNA Sequencing and Library Building Kit (E7645).

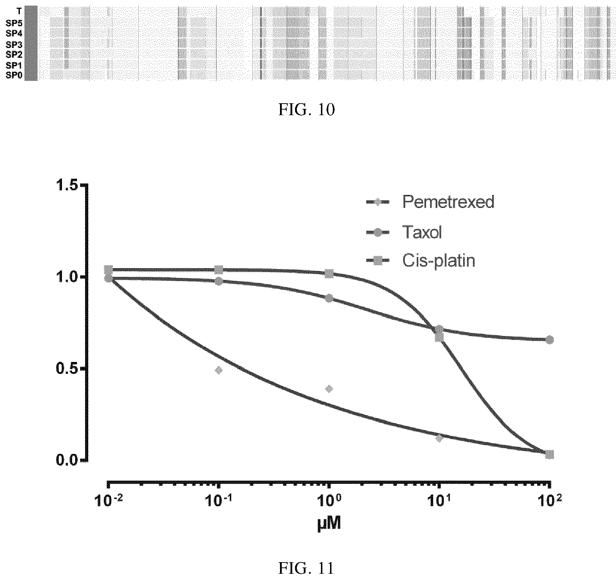

The high-throughput sequencing mentioned in the following embodiment refers to the Illumina HiSeq X-ten sequencing platform.
1. Pleural effusion samples of lung cancer were obtained, 20 mL of pleural effusion sample of lung cancer was firstly centrifugated before operations of in vitro culture taken, cell precipitation of pleural effusion was obtained for DNA extraction, library building and whole-genome high-throughput sequencing (WGS), and with a sequencing depth of 30× before in vitro culture operations were performed, the remaining pleural effusion samples of lung cancer were used for culturing in vitro primary tumor cells from pleural effusion of lung cancer (the specific method refers to that in the previously described embodiment).
2. The pleural effusion samples of lung cancer were treated and cultured with the culture medium for primary cells for pleural effusion samples of lung cancer (Table 8, wherein the final concentration of human recombinant protein EGF was 60 ng/mL; the final concentration of human recombinant protein bFGF was 30 ng/mL; the final concentration of human recombinant protein MSP was 25 ng/mL; the final concentration of cortisol was 40 ng/mL; and the final concentration of Y-27632 was 15 µM) for a period of time to form cell masses with a diameter of more than 100 µm, which were recorded as P0-generation cells, followed by P1, P2, ..., Pn successively according to the number of passages. 10⁶ cells were taken respectively from P1, P2, P3, P4 and P5-generation primary tumor cell cultures from pleural effusion of lung cancer for DNA extraction, library building and whole-genome high-throughput sequencing (WGS), with a sequencing depth of 30×.
3. Copy number variation analysis (CNV) was performed based on the sequencing results of each group, and the copy number variations between cancer cells in pleural effusion of lung cancer and all generations of primary cell cultures from pleural effusion of lung cancer were compared. As shown in FIG. 10, the copy number variation of all generations of primary cell cultures from pleural effusion of lung cancer (P1, P2, P3, P4 and P5) was highly consistent with that of the cancer cells in pleural effusion of lung cancer, so the primary tumor cells from pleural effusion of lung cancer obtained by the present method could represent the real condition of the cancer cells in patient's pleural effusion.

### Embodiment 28. Comparing success rates of different primary cell culture media for culturing primary tumor cells from pleural effusion of lung cancer

The operation methods and processes for culturing primary cells from all samples in this embodiment were identical (as mentioned above), and the only difference lied in the culture medium formula. Various tested primary cell culture media are shown in Table 30. Wherein Scheme D is the formula used in the present invention, with details in Table 8 (wherein the final concentration of human recombinant protein EGF was 60 ng/mL; the final concentration of human recombinant protein bFGF was 30 ng/mL; the final concentration of human recombinant protein MSP was 25 ng/mL; the final concentration of cortisol was 40 ng/mL; and the final concentration of Y-27632 was 15 µM).

**Table 30 Primary Cell Culture Medium Formula For Test (100 mL)**

| Culture Medium | Reagent | Brand Article No. | Dosage | Final Concentration |
|---|---|---|---|---|
| Scheme A | mTeSR^{™}1 medium | Stemcell#05850 | 100 mL | |
| Scheme B | Alveolar Epithelial Cell Medium | ScienCell#3201 | 100 mL | |
| Scheme C | Dual-antibody P/S | Gibco#15140122 | 1 mL | 1% |
| | HEPES | Gibco#15630080 | 1 mL | 10 mM |
| | 1,000× human recombinant protein | 1,000× stock solution | 250 µL | 50 ng/mL |
| | EGF 1,000× human recombinant protein bFGF | 1,000× stock solution | 100 µL | 20 ng/mL |
| | DMEM/F12 culture medium | Gibco# 14170161 | Added to 100 mL | |
| Scheme D | See Table 8 | | | |

After being prepared, the primary cell culture media were filtered and sterilized with a 0.22 µMsyringe-driven strainer (Millipore SLGP033RS). The primary cell culture media can be stored at 4 °C for two weeks.

20 pleural effusion samples of lung cancer were treated according to each of the four primary cell culture medium schemes, the sample treatment and culture operations were performed according to the methods described in embodiments 19, 20 and 21, and the success rates of those primary cell culture media for culturing primary tumor cells from pleural effusion of lung cancer were counted after 10 days of culturing, as shown in Table 31:

**Table 31 Culturing in different media**

| Primary cell culture medium scheme | Success rate |
|---|---|
| Scheme A | 0% (0/20) |
| Scheme B | 0% (0/20) |
| Scheme C | 20% (4/20) |
| Scheme D | 85% (17/20) |

It can be seen that primary cell culture media have an extremely great impact on the success rate of culturing primary cells from pleural effusion of lung cancer, and the culture medium for primary cells from pleural effusion of lung cancer used in the present invention (Table 8) can, to the greatest extent, stimulate the proliferation of cancer cells in the pleural effusion samples of lung cancer and improve the success rate of culturing primary tumor cells from pleural effusion of lung cancer.

### Embodiment 29. Comparing success rates of different cell digestion solutions for passaging primary tumor cells from pleural effusion of lung cancer

The operation methods and processes for passaging primary cells of all samples in this embodiment were identical (as mentioned above), and the only difference lied in the cell digestion solution formula. Various tested cell digestion solutions are shown in Table 32. Wherein Scheme D is the formula used in the present invention, and the details are shown in Table 6.

**Table 32 Cell Digestion Solution Formula for Test (10 mL)**

| Cell Digestion Solution | Reagent | Brand Article No. | Dosage | Final Concentration |
|---|---|---|---|---|
| Scheme A | Trypsin | Gibco# 252,00056 | 10 mL | |
| Scheme B | 0.5M EDTA | Invitrogen#AM9261 | 10 µL | 5 mM |
| | TrypLE^{™} Express | Gibco#12604013 | Added to 10 mL | |
| Scheme C | 10× collagenase I | 10× stock solution | 1 mL | 200 U/mL |
| | 10× collagenase II | 10× stock solution | 1 mL | 200 U/mL |
| | 20× collagenase IV | 20× stock solution | 0.5 mL | 100 U/mL |
| | PBS | Gibco#21-040-CVR | Added to 10 mL | |
| Scheme D | See Table 6 | | | |

The cell digestion solution needs to be prepared just before use.

20 successfully cultured pleural effusion samples of lung cancer were selected, and the primary tumor cells from pleural effusion of lung cancer obtained by culture were continuously passaged with the above four cell digestion solutions respectively according to the method described in Embodiment 22. Passaging (for no more than 10 times) was performed every time cancer cells amplified and formed cell masses with a diameter of 100 µm, and the maximum passaging number was recorded. The statistical results are as shown in Table 33:

**Table 33 Culturing in Different Cell Digestion Solutions**

| S/N | Sample No. | Scheme A | Scheme B | Scheme C | Scheme D |
|---|---|---|---|---|---|
| 1 | 20180912HEQ3227 | 0 | 2 | 5 | 9 |
| 2 | 20181029FJP6863 | 0 | 2 | 5 | 9 |
| 3 | 20181030YLM0526 | 1 | 3 | 6 | 10 |
| 4 | 20181109ZCY1117 | 0 | 0 | 2 | 10 |
| 5 | 20181112FSL8342 | 0 | 1 | 3 | 6 |
| 6 | 20181123RBH9525 | 1 | 3 | 7 | 10 |
| 7 | 20181130ZZC0128 | 0 | 1 | 3 | 6 |
| 8 | 20181224ZPB2339 | 0 | 2 | 6 | 10 |
| 9 | 20180513HB1783 | 0 | 2 | 4 | 8 |
| 10 | 20180606ZSD6376 | 0 | 2 | 5 | 10 |
| 11 | 20180607LYS8025 | 0 | 1 | 5 | 10 |
| 12 | 20180710LSW2025 | 1 | 4 | 7 | 10 |
| 13 | 20180724CRQ4027 | 0 | 0 | 3 | 6 |
| 14 | 20180830CDD0046 | 0 | 2 | 5 | 9 |
| 15 | 20180831HZM4008 | 0 | 1 | 4 | 7 |
| 16 | 20180904CRH4057 | 1 | 3 | 6 | 9 |
| 17 | 20180801YDH1008 | 1 | 2 | 5 | 10 |
| 18 | 20181126QSM6029 | 0 | 2 | 5 | 9 |
| 19 | 20180629LGQ0829 | 0 | 2 | 4 | 8 |
| 20 | 20180521LHF5893 | 0 | 2 | 7 | 10 |
| Summary of passaging number | 0-1 times | 0-4 times | 2-7 times | More than 6 times | |

It can be seen that different cell digestion solutions have a very great impact on the success rate of passaging primary tumor cells from pleural effusion of lung cancer, and the cell digestion solution used in the present invention (Table 6) can mildly dissociate the cancer cells in the cell masses to continuously passage the samples and maintain the activity of primary tumor cells from pleural effusion of lung cancer.

### Embodiment 30. Culturing primary tumor cells from pleural effusion of lung cancer with cell culture consumables of different materials

The operation methods and processes of primary culturing of all samples in this embodiment were identical (as mentioned above), and the only difference lied in the cell culture consumable material (unmodified) (Table 34).

**Table 34 Effects of Unmodified Culture Consumables of Different Materials on Culturing Primary Tumor Cells from Pleural Effusion of Lung Cancer**

| S/N | Sample No. | PS | PC | PMMA | COC | COP | LAS |
|---|---|---|---|---|---|---|---|
| 1 | 20180620YSF6868 | Passed | Passed | Passed | Passed | Passed | Passed |
| 2 | 20180521LSM1885 | Failed | Failed | Failed | Passed | Failed | Passed |
| 3 | 20180514ZCR1379 | Failed | Failed | Passed | Passed | Passed | Passed |
| 4 | 20181203HFL0708 | Failed | Failed | Passed | Passed | Failed | Passed |
| 5 | 20181201YXZ0503 | Failed | Failed | Failed | Failed | Failed | Failed |
| 6 | 20181117SXH1455 | Failed | Failed | Failed | Failed | Failed | Passed |
| 7 | 20181207AYD7020 | Failed | Failed | Failed | Failed | Failed | Passed |
| 8 | 20181024XQH1632 | Passed | Failed | Passed | Passed | Failed | Passed |
| 9 | 20181202XTN1215 | Failed | Failed | Failed | Failed | Failed | Passed |
| 10 | 20180606YB2673 | Passed | Passed | Passed | Passed | Passed | Passed |
| | Success Rate | 3/10 | 2/10 | 5/10 | 6/10 | 3/10 | 9/10 |

| | | | | | | | |
|---|---|---|---|---|---|---|---|
| Notes: Polystyrene (abbreviated as PS), Polycarbonate (abbreviated as PC), poly-methyl methacrylate (abbreviated as PMMA), COC resin, Cyclo Olefin Polymer (abbreviated as COP), low-attachment-surface (abbreviated as LAS). | | | | | | | |

It can be seen from Table 34 that culture consumables of different materials have a certain impact on the success rates for culturing primary tumor cells from pleural effusion of lung cancer, wherein the success rate of low-attachment-surface (LAS) is the highest.

### Embodiment 31. Culturing primary tumor cells from pleural effusion of lung cancer with CYTOP-modified cell culture consumables of different materials

The operation methods and processes of primary culturing of all samples in this embodiment were identical (as mentioned above), the CYTOP modification methods were identical, and the only difference lied in the cell culture consumable material (Table 35).

Wherein the CYTOP modification method was that: firstly, pure oxygen etching was performed based on the cell culture vessel at a power of 20 W power for 3 minutes. Then the surface of a culture dish or culture plate was covered with an appropriate amount (taking a 96-well plate as an example, with 20 µL per well, an appropriate amount refers to complete coverage of the bottom of the culture dish) of 1% CYTOP solution (see Table 36 for the formula), and the vessel could be used after the CYTOP solution was completely dried in the air.

**Table 35 Effects of CYTOP-modified Culture Consumables of Different Materials on Culturing Primary Tumor Cells from Pleural Effusion of Lung Cancer**

| S/N | Sample No. | PS | PC | PMMA | COC | COP | LAS |
|---|---|---|---|---|---|---|---|
| 1 | 20190104RBH9525 | Passed | Passed | Passed | Passed | Passed | Passed |
| 2 | 20190103QJF2041 | Failed | Failed | Failed | Failed | Failed | Failed |
| 3 | 20190103ZXH8882 | Passed | Passed | Passed | Passed | Passed | Passed |
| 4 | 20190215LY0508 | Passed | Passed | Passed | Passed | Passed | Passed |
| 5 | 20190606LBD0034 | Passed | Passed | Passed | Passed | Passed | Passed |
| 6 | 20190505FQ5060 | Passed | Passed | Passed | Passed | Passed | Passed |
| 7 | 20190428YJH1590 | Passed | Passed | Passed | Passed | Passed | Passed |
| 8 | 20190527KDY1723 | Failed | Failed | Failed | Failed | Failed | Failed |
| 9 | 20190425SSS1683 | Passed | Passed | Passed | Passed | Passed | Passed |
| 10 | 20190523ZYH2893 | Passed | Passed | Passed | Passed | Passed | Passed |
| | Success Rate | 8/10 | 8/10 | 8/10 | 8/10 | 8/10 | 8/10 |

It can be seen from Table 35 that: CYTOP modification can effectively improve the culture success rates of various materials.

**Table 36 1% CYTOP Solution (100 mL)**

| Reagent | Brand Article No. | Dosage | Final Concentration |
|---|---|---|---|
| CYTOP | Asashi | 1 mL | 1% |
| Fluorocarbon oil | glass#CTL-809M 3M# FC40 | Added to 100 mL | |

The prepared 1% CYTOP solution can be stored at a normal temperature for a long time.

### Example 32. Performing chemosensitivity assay with primary tumor cells from pleural effusion of lung cancer

All the chemotherapeutic agents used in this embodiment, such as Taxol, Pemetrexed and Cis-platin, are Selleck products.

The Celltiter-Glo cell viability test kit mentioned in this embodiment is a Promega product.

An in vitro chemosensitivity assay was performed for the primary tumor cells from pleural effusion of lung cancer cultured by the present invention: primary cells were inoculated at a density of 10⁵/well in a 96-well low-attachment cell culture plate of standard size, and 5 drug concentration gradients were set for each drug, n=3. After dosing, the cells were incubated under the condition of 37°C and 5% CO₂ for 7 days. Following the end of drug action, the cell viability in each well was detected by the Celltiter-Glo cell viability assay kit. The experimental results are as shown in FIG. 11. The results indicated that: the primary tumor cells from pleural effusion of lung cancer obtained by the present method can be used for in vitro chemosensitivity assay.

### Embodiment 33. Processing a microplate chip

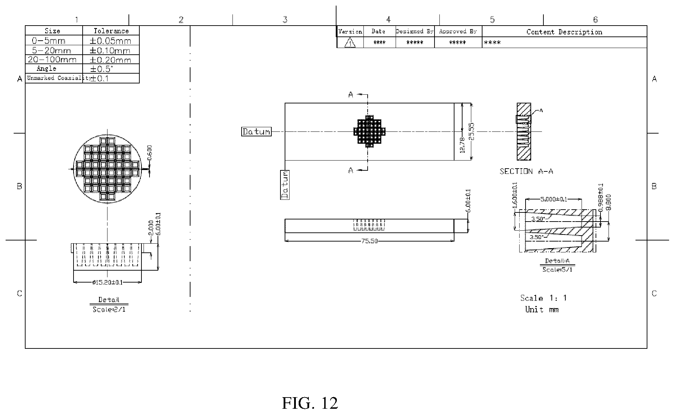

In the embodiment, a microplate chip used for culturing the primary cells of lung cancer in the present invention was made from PMMA material (or PS, PC, COC, COP, LAS and other materials) by injection molding. The chip can be used for culturing primary cells of lung cancer and conducting in vitro chemosensitivity assay. The design drawing of the microplate chip is shown in FIG. 12.

Specifically, in the process of practical application, the microplate chip structure (design drawing as shown in FIG. 12) was prepared from PMMA material (or PS, PC, COC, COP, LAS and other materials), then the surface of the microplate chip was CYTOP-modified by the above CYTOP modification method (see Embodiment 31), so that the microplate chip available for culturing the primary cells of lung cancer as stated herein was obtained.

### Embodiment 34. Culturing primary cells from lung cancer samples of various pathological types

Primary cells were cultured from the surgical samples, puncture samples and pleural effusion samples of non-small-cell lung cancer adenocarcinoma, non-small-cell lung cancer squamous cell carcinoma and small cell lung cancer by the previously described methods, and the cultures are as shown in Table 37.

**Table 37 Culturing Primary Cells from Lung Cancer Samples of Various Pathological Types**

| S/N | Sample No. | Culture (number of successful cases/number of total cases) |
|---|---|---|
| Adenocarcinoma | Surgical sample | 35/40 |
| | Puncture sample | 4/5 |
| | Pleural effusion sample | 33/40 |
| Squamous cell carcinoma | Surgical sample | 14/20 |
| | Puncture sample | 1/2 |
| | Pleural effusion sample | 5/7 |
| Small cell lung cancer | Surgical sample | 4/6 |
| | Puncture sample | 2/3 |
| | Pleural effusion sample | 7/10 |

It can be seen from Table 37 that all the lung cancer samples of various pathological types can be used to culture primary cells of lung cancer at a higher success rate.

### Industrial Application

The present invention provides a method for culturing primary tumor cells from fresh solid tumor tissues of lung cancer or pleural effusion of lung cancer and the auxiliary reagents. The method has the following advantages: for solid tumor tissues of lung cancer, the amount of the tissue sample required is small, and only about 20 mg of surgical sample of lung cancer is required. For pleural effusion of lung cancer, the sample is easy to obtain, and the pleural effusion discharged from patients with lung cancer during the conventional treatment is utilized to the greatest extent, and there's no additional trauma or pain for the patients; the amount of the sample required is small, and only about 50 mL of pleural effusion of lung cancer is required; there's no need to process the samples immediately after collection, and the present method can guarantee a cell viability of more than 90% for up to 72 hours after the sample is ex vivo. The culture cycle is short, and it only takes3-10 days to obtain the primary tumor cells with the order of magnitude of 10⁷. The culture stability is high, and the success rate of the method for culturing in vitro qualified surgical samples of lung cancer is as high as 70%-80%. The cell purity is high, and the proportion of cancer cells in the primary cell cultures of lung cancer obtained by the present method can reach up to 70%-95%, with less interference from miscellaneous cells. The primary cell cultures of lung cancer obtained by the method of the present invention can be used for various cell-level in vitro tests, next generation sequencing, construction of animal models, construction of cell lines and the like. It is predictable that this culture method will have a broad application prospect in the fields of lung cancer research, clinical diagnosis, and treatment.

## Claims

1. A medium for culturing primary cells of lung cancer, comprising a dual-antibody P/S, HEPES, a non-essential amino acid solution, GlutaMax, human recombinant protein EGF, human recombinant protein bFGF, human recombinant protein MSP, cortisol, B27, ITS-X, Y-27632 and an Advanced DMEM/F12 medium; wherein the final concentration of penicillin in the dual-antibody P/S is 100-200 U/mL; the final concentration of streptomycin in the dual-antibody P/S is 100-200 µg/mL the final concentration of the HEPES is 8-12 mM; the final concentration of the non-essential amino acid solution is 0.8-1.2% (volume percentage); the final concentration of the GlutaMax is 0.8-1.2% (volume percentage); the final concentration of the human recombinant protein EGF is 10-100 ng/mL; the final concentration of the human recombinant protein bFGF is 10-50 ng/mL; the final concentration of the human recombinant protein MSP is 5-25 ng/mL; the final concentration of the cortisol is 20-50 ng/mL; the final concentration of the B27 is 1.5-2.5% (volume percentage); the final concentration of the ITS-X is 0.8-1.2% (volume percentage); the final concentration of the Y-27632 is 5-20 µM; and the rest is the Advanced DMEM/F12 medium.

2. A reagent set for culturing primary cells of lung cancer, which is any of the followings:
(A1) Consisting of the culture medium of claim 1 and all or part of the following: sample dissociation solution, sample preservation solution and sample washing solution;
the sample dissociation solution is composed of collagenase I, collagenase IV and PBS; wherein the final concentration of the collagenase I in the sample dissociation solution is 150-250 U/mL; the final concentration of the collagenase IV in the sample dissociation solution is 150-250 U/mL; and the rest is PBS;
the sample preservation solution is composed of fetal bovine serum, dual-antibody P/S, HEPES and HBSS; wherein the final concentration of the fetal bovine serum in the sample preservation solution is 1-5% (volume percentage); the final concentration of the penicillin of the dual-antibody P/S in the sample preservation solution is 100-200 U/mL; the final concentration of the streptomycin of the dual-antibody P/S in the sample preservation solution is 100-200 µg/mL; the final concentration of the HEPES in the sample preservation solution is 8-12 mM; and the rest is HBSS;
the sample washing solution is composed of dual-antibody P/S and PBS; wherein the final concentration of the penicillin of the dual-antibody P/S in the sample washing solution is 100-200 U/mL; the final concentration of the streptomycin of the dual-antibody P/S in the sample washing solution is 100-200 µg/mL; and the rest is PBS;
(A2) Consisting of the culture medium of claim 1 and cell isolation buffer;
the cell isolation buffer is composed of dual-antibody P/S, heparin sodium and PBS; wherein the final concentration of the penicillin in the dual-antibody P/S is 100-200 U/mL U/mL; the final concentration of the streptomycin in the dual-antibody P/S is 100-200 µg/mL; the final concentration of the heparin sodium is 10 IU/mL; and the rest is PBS;
(A3) Consisting of (A1) and all or part of the following reagents: cell digestion solution, digestion termination solution and cell cryopreservation solution; and
(A4) Consisting of (A2) and all or part of the following reagents: cell digestion solution, digestion termination solution and cell cryopreservation solution;
the composition of the cell digestion solution is as follows: each 10 mL of the cell digestion solution contains 4-6 mL of Accutase, EDTA with a final concentration of 5 mM and 1.5-2.5 mL of TrypLE Express, and the rest is PBS;
the sample preservation solution is composed of fetal bovine serum, dual-antibody P/S and a DMEM medium; wherein the final concentration of the fetal bovine serum in the digestion termination solution is 8-12% (volume percentage); the final concentration of the penicillin of the dual-antibody P/S in the digestion termination solution is 100-200 U/mL; the final concentration of the streptomycin of the dual-antibody P/S in the digestion termination solution is 100-200 µg/mL; and the rest is the DMEM medium;
the cell cryopreservation solution is composed of an Advanced DMEM/F12 medium, DMSO and 1% methylcellulose solution; wherein the volume ratio of the Advanced DMEM/F12 medium, the DMSO and the 1% methylcellulose solution is 20:2:(0.8-1.2); and the 1% methylcellulose solution is an aqueous solution of methylcellulose with a concentration of 1 g/100 ml.

3. Any of the following applications:
(B1) an application of the culture medium of claim 1 in culturing primary cells of lung cancer;
(B2) an application of the reagent set described in (A1) or (A3) of claim 2 in culturing primary cells from solid tumor of lung cancer; and
(B3) an application of the reagent set described in (A2) or (A4) of claim 2 in culturing primary tumor cells from pleural effusion of lung cancer.

4. A method for culturing primary cells of lung cancer, which is either method A or method B:
method A: a method for culturing primary cells from solid tumor tissues of lung cancer, comprising the following steps:
(a1) dissociating solid tumor tissues of lung cancer with the sample dissociation solution described in claim 2, to obtain primary cells from solid tumor of lung cancer;
(a2) suspension-culturing the dissociated primary cells from solid tumor of lung cancer in step (a1) with the culture medium of claim 1;
method B: a method for culturing primary tumor cells from pleural effusion of lung cancer, comprising the following steps:
(b1) separating primary tumor cells from pleural effusion of lung cancer, to obtain primary tumor cells from pleural effusion of lung cancer;
(b2) suspension-culturing the separated primary tumor cells from pleural effusion of lung cancer in step (b1) with the culture medium of claim 1.

5. The method according to claim 4, wherein in step (a1), the solid tumor tissues of lung cancer are dissociated with the sample dissociation solution according to a method comprising the following steps: according to the dosage of 0.1-0.3 mL of sample dissociation solution per 1 mg of tissue, treating the solid tumor tissues of lung cancer, which were cut up, with the sample dissociation solution preheated to 37°C in advance, and dissociating the sample at 37°C for 15 minutes to 3 hours;
in step (b1), the primary tumor cells from pleural effusion of lung cancer can be separated from the pleural effusion of lung cancer according to a method comprising the following steps: suspending the cells in the pleural effusion of lung cancer with the cell isolation buffer described in claim 2, and then obtaining the primary tumor cells from pleural effusion of lung cancer by density gradient centrifugation.

6. The method according to claim 4 or 5, wherein in step (a2), the primary cells from solid tumor of lung cancer are suspension-cultured with the culture medium according to a method comprising the following steps: using a cell culture vessel M, suspension-culturing the primary cells from solid tumor of lung cancer with the culture medium, under the condition of 37°C and 5% CO₂, and replacing the culture medium every 2-4 days;
in the step (b2), the primary tumor cells from pleural effusion of lung cancer are suspension-cultured with the culture medium according to a method comprising the following steps: using a cell culture vessel M, suspension-culturing the primary tumor cells from pleural effusion of lung cancer with the culture medium, under the condition of 37°C and 5% CO₂, and replacing the culture medium every 2-4 days;
the cell culture vessel M is any of the followings: (I) a cell culture vessel made of polystyrene, a cell culture vessel made of polycarbonate, a cell culture vessel made of polymethylmethacrylate, a cell culture vessel made of COC resin, a cell culture vessel made of cycloolefin polymer or a cell culture vessel with a low adsorption surface; (II) a cell culture vessel after CYTOP modification of the cell culture vessel in (I).

7. The method according to claim 6, wherein in (II), the cell culture vessel in (I) is CYTOP-modified according to a method comprising the following steps: performing pure oxygen etching on the cell culture vessel in (I) at an etching power of 20 W for 3 minutes; then covering the surface of the cell culture vessel with 1% CYTOP solution, and drying the 1% CYTOP solution in the air to complete CYTOP modification;
the composition of the 1% CYTOP solution is as follows: each 100 mL of the 1% CYTOP solution contains 1 mL of CYTOP, and the rest is fluorocarbon oil.

8. The method according to any of claims 4-7, wherein before step (a1), the following steps for predissociation treatment of the solid tumor tissues of lung cancer can also be included: washing the surface of a solid tumor tissue sample of lung cancer with 70-75% ethanol (by volume); and washing the solid tumor tissue sample of lung cancer with the sample washing solution and sterile PBS solution of claim 2 successively.

9. The method according to claim 8, wherein the in vitro time of the solid tumor tissue sample of lung cancer subject to predissociation treatment is within 2 hours, and the solid tumor tissue sample of lung cancer is preserved in the sample preservation solution described in claim 2 before the predissociation treatment.

10. The method according to any of claims 4-9, wherein in step (a1), the following steps are performed after dissociation treatment of the solid tumor tissues of lung cancer with the sample dissociation solution: terminating a dissociation reaction with the digestion termination solution described in claim 2, and collecting a cell suspension; filtering the cell suspension to remove tissue debris and adherent cells; centrifuging and then resuspending cells with sterile PBS; centrifuging again and resuspending the cell precipitation with the culture medium of claim 1.

11. The method according to any of claims 4-10, wherein before step (a2), the following step is also included: passaging the primary cells from solid tumor of lung cancer when masses with a diameter of 80-120 µm are formed by the primary cells from solid tumor of lung cancer;
in step (b2), the following step is also included: passaging the primary tumor cells from pleural effusion samples of lung cancer when masses with a diameter of 80-120 µm are formed by the primary tumor cells from pleural effusion samples of lung cancer.

12. The method according to claim 11, wherein the cell digestion solution for the passage is the cell digestion solution described in claim 2.

13. The method according to claim 11 or 12, wherein the digestion termination solution for the passage is the digestion termination solution described in claim 2

14. The method according to any of claims 4-13, wherein the method also comprises a step of cryopreserving and/or resuscitating the primary cells from solid tumor of lung cancer or the primary tumor cells from pleural effusion of lung cancer after 2-3 passages and amplifications;
the cell cryopreservation solution used for the cryopreservation is the cell cryopreservation solution described in claim 2.

15. Any of the following reagents:
(C1) sample dissociation solution of solid tumor tissues of lung cancer, which is the sample dissociation solution described in claim 2;
(C2) sample preservation solution of solid tumor tissues of lung cancer, which is the sample preservation solution described in claim 2;
(C3) cell isolation buffer for cells from pleural effusion of lung cancer, which is the cell isolation buffer described in claim 2; and
(C4) cell digestion solution for primary cells of lung cancer, which is the cell digestion solution described in claim 2.

16. Any of the following applications:
(D1) an application of the sample dissociation solution described in (C1) in claim 15 in dissociating the primary cells of solid tumor of lung cancer from the solid tumor tissues of lung cancer;
(D2) an application of the sample preservation solution described in (C2) in claim 15 in preserving the solid tumor tissues of lung cancer;
(D3) an application of the cell isolation buffer described in (C3) in claim 15 in isolating the primary tumor cells of pleural effusion of lung cancer from the pleural effusion of lung cancer; and
(D4) an application of the cell digestion solution described in (C4) in claim 15 in passaging primary cells of lung cancer.

17. Any of the following methods:
(E1) a method for dissociating primary cells of solid tumor of lung cancer from the solid tumor tissues of lung cancer, comprising step (a1) in any of claim 4-14;
(E2) a method for preserving solid tumor tissues of lung cancer, comprising the following steps: preserving the solid tumor tissues of lung cancer just detached in the sample preservation solution described in claim 2 for no more than 2 hours;
(E3) a method for separating primary tumor cells in pleural effusion of lung cancer from the pleural effusion of lung cancer, comprising step (b1) in any of claim 4-14; and
(E4) a method for passaging primary cells of lung cancer, comprising the following steps: passaging the primary cells of lung cancer when masses with a diameter of 80-120 µm are formed by the primary cells of lung cancer; the cell digestion solution used for passaging is the cell digestion solution described in claim 2; the digestion termination solution used for passaging is the digestion termination solution described in claim 2.

18. The culture medium or reagent set or application or method according to any of claims 1-17, wherein the lung cancer is primary lung cancer.

19. The culture medium or reagent set or application or method according to any of claims 1-18, wherein the lung cancer is non-small-cell lung cancer or small cell lung cancer.

20. The culture medium or reagent set or application or method according to claim 19, wherein the non-small-cell lung cancer is non-small-cell lung cancer adenocarcinoma or non-small-cell lung cancer squamous cell carcinoma.

21. The culture medium or reagent set or application or method according to any of claims 1-20, wherein the primary cells of lung cancer are primary cells from solid tumor of lung cancer or primary tumor cells from pleural effusion of lung cancer.

22. The culture medium or reagent set or application or method according to any of claims 1-21, wherein the primary cells of lung cancer are isolated from surgical samples, puncture samples or pleural effusion samples of patients with lung cancer.
